# EUROPEAN PATENT SPECIFICATION

(11) **EP 2 831 584 B1**
(45) Date of publication and mention of the grant of the patent: **17.06.2020**
(21) Application number: 13767635.9
(22) Date of filing: 21.03.2013
(51) Int. Cl.: G01N 33/50, G01N 33/569

(54) **CELLULAR MARKERS FOR DIAGNOSIS OF ALZHEIMER'S DISEASE AND FOR ALZHEIMER'S DISEASE PROGRESSION**
ZELLULÄRE MARKER ZUR DIAGNOSE VON MORBUS ALZHEIMER UND DES FORTSCHREITENS VON MORBUS ALZHEIMER
MARQUEURS CELLULAIRES POUR DIAGNOSTIQUER LA MALADIE D'ALZHEIMER ET LA PROGRESSION DE LA MALADIE D'ALZHEIMER

(30) Priority: 26.03.2012 US 201261615465 P
(43) Date of publication of application: 04.02.2015
(73) Proprietor: Yeda Research and Development Co., Ltd., 7610002 Rehovot (IL); Neuroquest Ltd., 20179 Misgav (IL)
(72) Inventor: EISENBACH-SCHWARTZ, Michal, 76100 Rehovot (IL); YOLES, Ester, 76880 (IL)
(74) Representative: Lavoix
(86) International application number: PCT/IL2013/050277
(87) International publication number: WO 2013/144957

(56) References cited:
- WO-A1-2011/111043
- WO-A1-2011/111043
- US-A1- 2002 137 108
- FISZER U: "Role of gamma-delta T-cells and heat shock proteins in immunological response and in pathogenesis of neurological diseases", NEUROLOGIA I NEUROCHIRURGIA POLSKA, POLSKIE TOWARZYSTWO NEUROLOGICZNE, WARSAW, PL, vol. 29, no. 5, 1 January 1995 (1995-01-01), pages 737-745, XP008175022, ISSN: 0028-3843
- FISZER U: 'Role of gamma-delta T-cells and heat shock proteins in immunological response and in pathogenesis of neurological diseases.' NEUROL NEUROCHIR POL vol. 29, no. 5, 1995, page 737, XP008175022 Retrieved from the Internet: <URL:http://europepmc.org/abstract/MED/8584 100> [retrieved on 1995-10-31]

## Description

### TECHNICAL FIELD

The present invention relates to methods for early diagnosis of Alzheimer's disease.

### BACKGROUND ART

Neurodegenerative diseases such as Alzheimer's disease (AD), Parkinson's disease or amyotrophic lateral sclerosis (ALS) share a chronic progressive course that leads to neurodegeneration, including neuroaxonal damage, apoptosis and gliosis. The pathogenesis and pathophysiology of neurodegenerative diseases are extremely complex and only partially understood. Regardless of the differences, age is a common risk factor that plays a significant role in the pathophysiology of neurodegenerative diseases. In addition, there is substantial evidence that excitotoxicity, oxidative stress, protein aggregation, inflammation and apoptosis, among others, are common pathological events that have a role in disease progression. After more than two decades of intensive efforts by scientific and pharmaceutical communities throughout the world and despite of the accumulating knowledge, neurodegenerative diseases are still unpreventable, incurable and largely untreatable. Furthermore, no objective test is available for definitive diagnosis. Diagnosis is typically done using clinical assessments at advanced stages of the disease when damage is significant and potential for delaying disease progression is low.

The immune system, by employing an array of cellular network, is the body's natural mechanism for host defense against foreign entities as well as for tissue maintenance, healing, regeneration and surveillance against aberrant cell growth, i.e., the recognition of tumors or transformed cells. Yet any activity of the peripheral immune cells in the central nervous system (CNS) was long considered to be undesirable. The CNS of vertebrate animals is uniquely protected from toxins, invading pathogens, inflammatory cells and macromolecules through a protective mechanism called the "blood-brain barrier", a system of tight junctions at capillaries within the CNS that provides a protective physical barricade. Under normal non-pathological conditions, blood-borne immune cells can barely be detected in the brain using standard histological methods. The scarcity of blood-borne immune cells in healthy CNS parenchyma, in combination with the concept of the CNS being an "immune privileged site", contributed to the common view that, under normal conditions, the CNS functions most effectively in the absence of any immune cell activity.

In contrast to the common consideration, it has recently become evident that the nervous and immune systems are engaged in an intense bidirectional communication. Immune cells were also found to have a role in the different steps of neurogenesis including progenitor proliferation, survival, migration and differentiation (Ziv and Schwartz, 2008; Ekdahl *et al.*, 2008).

Active T cells were shown to patrol the CNS at all times under both normal and pathological conditions, while animals deprived of activated T cells show reduced memory capabilities which can be reversed by replenishment with T cells (Butovsky *et al.*, 2006a; Kipnis *et al.*, 2004; Ziv *et al.*, 2006).

The positive role of auto-reactive T cells in maintaining the normal activity of the brain in normal and pathological conditions was described in various publications (Schwartz, 2001; Schori *et al.*, 2001; Mizrahi *et al.*, 2002; Nevo *et al.*, 2003; Nevo *et al.*, 2004). In non-pathological conditions, it is suggested that brain activity, such as intensive learning activity, involves continuing support from autoreactive T cells needed for restoration of homeostasis. Such T cells are located at the borders of the brain. At "the borders of the CNS", CNS-specific T cells become activated, secret cytokines and growth factors and also directly affect the microglia to become supportive to neuronal survival and growth.

In the injured CNS, an emerging understanding of the role of the immune system in regulating neurotoxicity by the secretion of growth factors, removal of dying neurons and detoxification of the environment, has suggested that the situation is complex, with a balance between beneficial and detrimental effects of the immune system (Shaked *et al.*, 2005; Shaked *et al.*, 2004; Ziv *et al.*, 2006; Kipnis *et al.*, 2004; Ron-Harel and Schwartz, 2009).

As further shown, in response to acute injury, effector T-cells (T-eff) directed to self-antigens (autoimmune T-cells) are needed as part of a reparative response (Rapalino *et al.*, 1998; Hauben *et al.*, 2000; Hauben *et al.*, 2003; Schwartz and Hauben, 2002; Moalem *et al.*, 1999; Yoles *et al.*, 2001; Kipnis *et al.*, 2001; Schwartz *et al.*, 2003), yet this activity should be tightly regulated by regulatory T cells (T-reg) (Taams and Akbar, 2005) as part of a mechanism to control autoimmune disease (Kipnis *et al.*, 2002; Schwartz and Kipnis, 2002; Schwartz and Kipnis, 2004; Kipnis and Schwartz, 2005). Accordingly, boosting autoimmunity was shown to be beneficial in animal models of acute or chronic neurodegenerative disorders.

Age-dependent decline in immunity, known as immunosenescence, is associated with reduced host defense, manifested by an increased susceptibility to infection diseases, as well as reduced ability to develop immunity after vaccination (Aw *et al.*, 2007). The decline in immunity in the elderly has largely been attributed to changes in hematopoietic stem cells function and their ability to differentiate into different immune cell lineages. This goes in line with the fact that the thymus involutes with age, so that the number of naive cells available to respond to new foreign antigens also declines. However, much less attention has been devoted to the role of the immune system in tissue maintenance, healing and regeneration. This is particularly important for understanding the link between brain aging, memory deterioration and immune senescence.

AD is an age related disease. In an animal model of AD, augmenting the adaptive immune response using glatiramer acetate vaccination resulted in decreased plaque formation and induction of neurogenesis (Butovsky *et al.*, 2006b). This treatment induced the recruitment of blood-borne monocytes to the diseased brain. Depletion of these blood-borne monocytes from the blood resulted in a significantly increased formation of amyloid plaques (Butovsky *et al.*, 2007). Furthermore, using the same animal model, exercises were shown to induce T-cell response which coincides with a decrease in amyloid plaques in advanced pathological states (Nichol *et al.*, 2008). Another subset of immune-cells shown to be involved with plaque formation are the naturally occurring CD4⁺CD25⁺ regulatory T cells. Neuronal loss caused by intraocular injection of aggregated beta-amyloid was significantly greater in immunodeficient mice than in normal mice. The neurodegeneration was attenuated or augmented by elimination or addition, respectively, of naturally occurring CD4⁺CD25⁺ regulatory T cells (Treg) (Avidan *et al.*, 2004).

It is suggested that immunity recognizing self-proteins residing in the brain provides a mechanism that can sense and respond to various deviations from CNS homeostasis and maintain tissue integrity (Schwartz and Ziv, 2008a; Schwartz and Ziv, 2008b). Accordingly, during old age, when the need for maintenance increases, the senescent immune system fails to provide the support required. Neurodegenerative diseases might emerge when the levels/potency of key immunological components, involved with anti-self response, reach threshold levels.

WO 2011/111043 discloses methods for early diagnosis of ALS and for monitoring ALS progression, utilizing cellular blood markers. In a particular method disclosed, the levels of gamma-delta T-cells, CD11b⁺/CD14⁻ cells, Lin⁻/DR⁻/CD33⁺ cells and CD14⁺/CD16⁺ cells in a peripheral blood sample of a tested individual are measured and compared with the range levels of each one of these cell types in blood samples of age-matched controls, wherein no change in the level of CD14⁺/CD16⁺ cells and increase in the levels of each one of the other cell types indicate that said individual has a higher likelihood of having ALS than said age-matched controls.

US 2002/137108 discloses CD45 isoform alteration in T-helper cells as a diagnostic marker in Alzheimer's disease.

Fiszer et al., 1995, NEUROLOGIA I NEUROCHIRURGIA POLSKA, POLSKIE TOWARZYSTWO NEUROLOGICZNE, WARSAW, PL, 29(5):737-745 discloses that gamma-delta T cells participate in the pathogenesis of neurological diseases, including AD.

### SUMMARY OF INVENTION

It has been found, in accordance with the present invention, that while no differences were observed in the amount of lymphocytes and monocytes in the blood of Alzheimer's patients, ALS patients and healthy volunteers, significant differences in sub-population of lymphocytes and monocytes typically involved with regulation of the adaptive immune response were observed in Alzheimer's patients. Particular such differences were found in the level of gamma-delta (γδ)-T cells, which were significantly elevated in Alzheimer's patients (although less than in ALS patients) in comparison to healthy controls, and the pro-inflammatory sub-set of monocytes CD14⁺/CD16⁺, which were remarkably elevated in Alzheimer's patients but not in ALS patients. Furthermore, while a dramatic elevation was found in the percentage of monocytes having the markers CD14⁻/CD11b⁺/CD15⁺, a phenotype associated with myeloid-derived suppressor cells (MDSCs), in the blood of ALS patients, no difference in the percentage of these cells was found between Alzheimer's patients and healthy controls.

In one aspect, the present invention thus relates to a method for diagnosing the likelihood of AD in a tested individual, said method comprising:
(i) measuring the levels of γδ T-cells and at least one cell type of MDSCs in a peripheral blood sample obtained from said individual; and
(ii) comparing the levels measured in (i) with reference levels representing range levels of γδ T-cells and said at least one cell type of MDSCs, respectively, in blood samples of age-matched controls, thus obtaining a profile expressing the levels measured in (i) relative to said reference levels, respectively,
wherein an increase in the level of γδ T-cells; and no change in the level of each one of said at least one cell type of MDSCs indicate that said individual has a higher likelihood of having AD than said age-matched controls.

In certain embodiments, this method further comprises measuring in step (i) the level of at least one cell type of pro-inflammatory monocytes in said blood sample; and comparing in step (ii) the level of said at least one cell type of pro-inflammatory monocytes with a reference level representing a range level of said at least one cell type of pro-inflammatory monocytes in blood samples of age-matched controls, wherein an increase in the level of γδ T-cells; no change in the level of each one of said at least one cell type of MDSCs; and an increase in the level of at least one of said at least one cell type of pro-inflammatory monocytes indicate that said individual has a higher likelihood of having AD than said age-matched controls.

In a further aspect, the present invention provides the use of a kit in diagnosing the likelihood of AD in a tested individual, said kit comprising:
(i) antibodies against each one of cell types selected from gamma-delta (γδ) T-cells and at least one cell type of myeloid derived suppressor cells (MDSCs); and
(ii) reagents for detecting said antibodies.

In certain embodiments, the cell types comprised within the kit of the invention further include at least one cell type of pro-inflammatory monocytes.

### BRIEF DESCRIPTION OF DRAWINGS

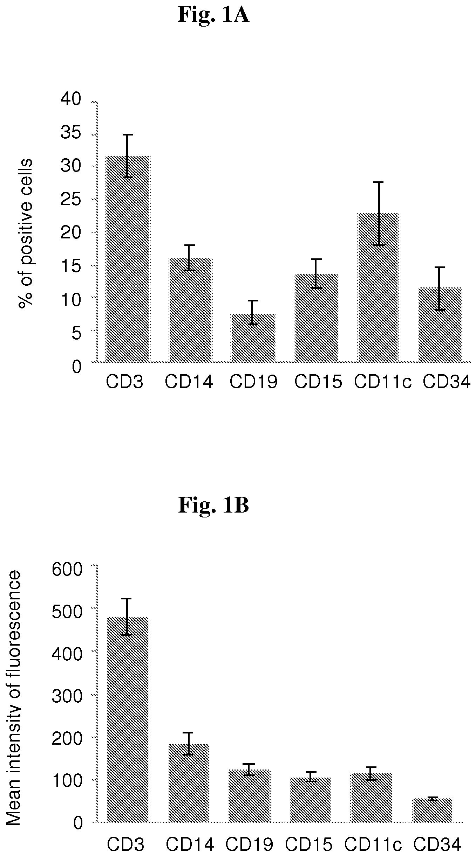

**Figs. 1A-1B** show distribution of single markers on total live PBMC. Freshly isolated PBMC of healthy volunteers were stained with FITC, PE or APC-labeled mononuclear antibodies against CD3, CD14, CD19, CD15, CDllc and CD34. The proportion (% of positive cells; **1A**) and level of expression (mean intensity of fluorescence; **1B**) of each marker was analyzed by FACS. Data shown are mean ± standard error (SE) from 4-6 different blood samples.

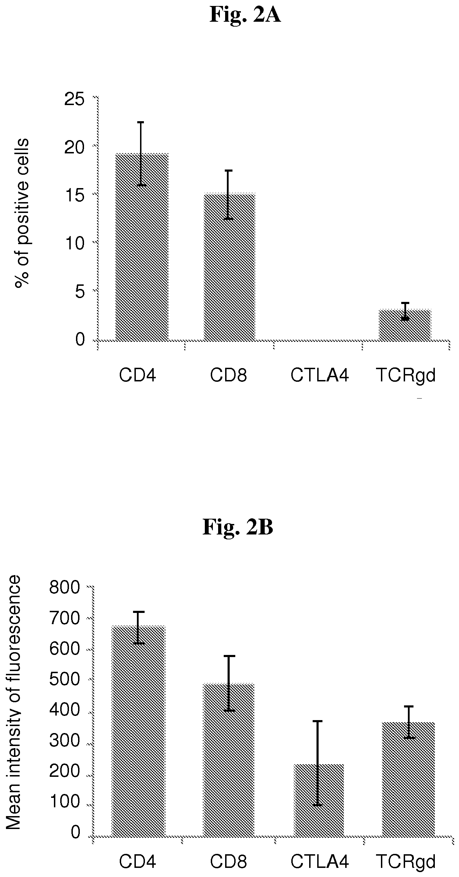

**Figs. 2A-2B** show lymphocyte sub-population. Freshly isolated PBMC of healthy volunteers were double stained with APC-labeled mononuclear antibodies against CD3 and one of the following FITC- or PE-labeled mononuclear antibodies against CD4, CD8, CTLA4 or TCRgd. Bars represents mean ± standard error (SE) of percentage of cells that express each of the markers out of the CD3 positive cell population (**2A**) and the intensity of expression (**2B**). Data shown are from 4 different experiments.

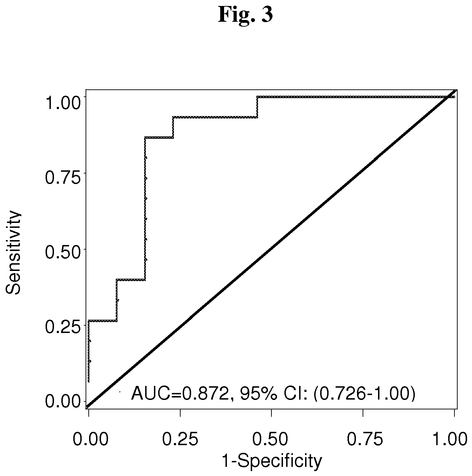

**Fig. 3** shows the receiver operator characteristic (ROC) curve for γδ T-cells analyzed based on the results obtained in the study described in Example 2, suggesting that the percentage of γδ T-cells out of total lymphocytes and monocytes appears to be highly sensitive and accurate for AD diagnosis.

### DETAILED DESCRIPTION OF THE INVENTION

The present invention provides a new approach identifying the age-related peripheral immune changes as primary risk factors for development of AD.

Preliminary studies conducted in accordance with the present invention and described hereinafter have shown specific and consistent changes, more particularly increase, in the levels of γδ T-cells and the pro-inflammatory monocytes CD14⁺/CD16⁺ cells in peripheral blood samples of Alzheimer's patients, compared with those measured in peripheral blood samples of age-matched controls, whereas no alteration has been observed in the level of the MDSCs CD11b⁺/CD14⁻/CD15⁺. This pattern of alterations is substantially different than that disclosed in the aforesaid WO 2011/111043 as indicative for ALS, wherein no change in the level of CD14⁺/CD16⁺ cells and increase in the levels of various MDSCs were clearly observed in peripheral blood samples of ALS patients. These findings indicate that specific changes in the level of certain T-cell or monocyte subsets such as those mentioned above can be used, either separately or in combination with each other or with other markers, as blood markers for diagnosis of AD.

In one aspect, the present invention thus relates to a method for diagnosing the likelihood of AD in a tested individual, said method comprising:
(i) measuring the levels of γδ T-cells and at least one cell type of MDSCs in a peripheral blood sample obtained from said individual; and
(ii) comparing the levels measured in (i) with reference levels representing range levels of γδ T-cells and said at least one cell type of MDSCs, respectively, in blood samples of age-matched controls, thus obtaining a profile expressing the levels measured in (i) relative to said reference levels, respectively,
wherein an increase in the level of γδ T-cells; and no change in the level of each one of said at least one cell type of MDSCs indicate that said individual has a higher likelihood of having AD than said age-matched controls.

The term "gamma-delta T-cells" (γδ T-cells), as used herein, refers to a small subset of T cells possessing a distinct T cell receptor (TCR) on their surface. In contrast to a majority of T cells in which the TCR is composed of two glycoprotein chains designated α- and β- TCR chains, the TCR in γδ T cells is made up of a γ-chain and a δ-chain. These cells were shown to play a role in immunosurveillance and immunoregulation (Girardi, 2006), and were found to be an important source of IL-17 (Roark *et al.*, 2008) and to induce robust CD8⁺ cytotoxic T cell response (Brandes *et al.*, 2009).

The term "myeloid derived suppressor cells" (MDSCs), as used herein, refers to a heterogeneous population of cells consisting of myeloid progenitor cells and immature myeloid cells (IMCs). In healthy individuals, IMCs that are quickly generated in the bone marrow differentiate into mature granulocytes, macrophages or dendritic cells (DCs). Interference with the differentiation of IMCs into mature myeloid cells results in the expansion of MDSC population. Accumulating evidence has shown that MDSCs contribute to the negative regulation of immune responses during cancer and other diseases. In human cancer, a subset of myeloid cells was found to have significantly increased arginase activity, which down-regulates expression of the T cell receptor CD3-ζ chain; and to suppress T cell proliferation, suggesting that these cells may mediate tumor-related immune suppression (Ochoa *et al.*, 2007; Zea *et al.*, 2005). Moreover, since it was shown that IL-13 plays a crucial role in MDSC suppressive activity (Beers *et al.*, 2008), our suggestion that MDSC activity is involved in disease progression is consistent with a report showing that the percentages of both CD4⁺IL-13⁺ and CD8⁺IL-13⁺ T cells in the blood of ALS patients are significantly higher than in healthy controls. The proportion of CD4⁺IL-13⁺ T cells was shown to have a significant negative correlation with the ALS functional rating scale scores, and a significant positive correlation with the rate of disease progression (Chiu *et al.*, 2008).

Non-limiting examples of MDSCs include CD11b⁺/CD14⁻, CD11b⁺/CD14⁻ /CD15⁺, CD11b⁺/CD14⁺/CD15⁺, Lin⁻/DR⁻, Lin⁻/DR⁻/CD33⁺, CD34⁺/CD33⁺/CD13⁺, ARG⁺/CD14⁺, CD34⁺/Lin⁻/DK/CD11b⁺/CD15⁺, CD14⁺/HLA-DR⁻/low, and Lin⁻/HLA-DR⁻ /low/CD11b⁺/CD33⁺ cell types.

In certain embodiments, the cells whose levels are measured in step (i) of the method of the invention are γδ T-cells and any one of CD11b⁺/CD14⁻, CD11b⁺/CD14⁻ /CD15⁺, CD11b⁺/CD14⁺/CD15⁺, Lin⁻/DR⁻, Lin/DR⁻/CD33⁺, CD34⁺/CD33⁺/CD13⁺, ARG⁺/CD14⁺, CD34⁺/Lin⁻/DR⁻/CD11b⁺/CD15⁺, CD14⁺/HLA-DR⁻/low, or Lin/HLA-DR⁻ /low/CD11b⁺/CD33⁺.

In certain embodiments, the cells whose levels are measured in step (i) of the method of the invention are γδ T-cells and any two cell types of the MDSCs listed, i.e., CD11b⁺/CD14⁻ and CD11b⁺/CD14⁻/CD15⁺; CD11b⁺/CD14⁻ and CD11b⁺/CD14⁺/CD15⁺; CD11b⁺/CD14⁻ and Lin⁻/DR⁻; CD11b⁺/CD14⁻ and Lin⁻/DR⁻/CD33⁺; CD11b⁺/CD14⁻ and CD34⁺/CD33⁺/CD13⁺; CD11b⁺/CD14⁻ and ARG⁺/CD14⁺; CD11b⁺/CD14⁻ and CD34⁺/Lin⁻ /DR⁻/CD11b⁺/CD15⁺; CD11b⁺/CD14⁻ and CD14⁺/HLA-DR/low; CD11b⁺/CD14⁻ and Lin⁻ /HLA-DR⁻/low/CD11b⁺/CD33⁺; CD11b⁺/CD14⁻/CD15⁺ and CD11b⁺/CD14⁺/CD15⁺; CD11b⁺/CD14⁻/CD15⁺ and Lin/DR⁻; CD11b⁺/CD14⁻/CD15⁺ and Lin/DR/CD33⁺; CD11b⁺/CD14⁻/CD15⁺ and CD34⁺/CD33⁺/CD13⁺; CD11b⁺/CD14⁻/CD15⁺ and ARG⁺/CD14⁺; CD11b⁺/CD14⁻/CD15⁺ and CD34⁺/Lin⁻/DR⁻/CD11b⁺/CD15⁺; CD11b⁺/CD14⁻/CD15⁺ and CD14⁺/HLA-DR⁻/low; CD11b⁺/CD14⁻/CD15⁺ and Lin⁻/HLA-DR⁻/low/CD11b⁺/CD33⁺; CD11b⁺/CD14⁺/CD15⁺ and Lin⁻/DR⁻; CD11b⁺/CD14⁺/CD15⁺ and Lin⁻/DR⁻/CD33⁺; CD11b⁺/CD14⁺/CD15⁺ and CD34⁺/CD33⁺/CD13⁺; CD11b⁺/CD14⁺/CD15⁺ and ARG⁺/CD14⁺; CD11b⁺/CD14⁺/CD15⁺ and CD34⁺/Lin⁻/DR⁻ /CD11b⁺/CD15⁺; CD11b⁺/CD14⁺/CD15⁺ and CD14⁺/HLA-DR⁻/low; CD11b⁺/CD14⁺/CD15⁺ and Lin⁻/HLA-DR⁻/low/CD11b⁺/CD33⁺; Lin⁻/DR⁻ and Lin⁻/DR⁻ /CD33⁺; Lin⁻/DR⁻ and CD34⁺/CD33⁺/CD13⁺; Lin⁻/DR⁻ and ARG⁺/CD14⁺; Lin⁻/DR⁻ and CD34⁺/Lin⁻/DR⁻/CD11b⁺/CD15⁺; Lin⁻/DR⁻ and CD14⁺/HLA-DR⁻/low; Lin⁻/DR⁻ and Lin⁻ /HLA-DR⁻/low/CD11b⁺/CD33⁺; Lin⁻/DR⁻/CD33⁺ and CD34⁺/CD33⁺/CD13⁺; Lin⁻/DR⁻ /CD33⁺ and ARG⁺/CD14⁺; Lin⁻/DR⁻/CD33⁺ and CD34⁺/Lin⁻/DR⁻/CD11b⁺/CD15⁺; Lin⁻ /DR⁻/CD33⁺ and CD14⁺/HLA-DR⁻/low; Lin⁻/DR⁻/CD33⁺ and Lin⁻/HLA-DR⁻ /low/CD11b⁺/CD33⁺; CD34⁺/CD33⁺/CD13⁺ and ARG⁺/CD14⁺; CD34⁺/CD33⁺/CD13⁺ and CD34⁺/Lin⁻/DR⁻/CD11b⁺/CD15⁺; CD34⁺/CD33⁺/CD13⁺ and CD14⁺/HLA-DR⁻/low; CD34⁺/CD33⁺/CD13⁺ and Lin⁻/HLA-DR⁻/low/CD11b⁺/CD33⁺; ARG⁺/CD14⁺ and CD34⁺/Lin⁻/DR⁻/CD11b⁺/CD15⁺; ARG⁺/CD14⁺ and CD14⁺/HLA-DR⁻/low; ARG⁺/CD14⁺ and Lin⁻/HLA-DR⁻/low/CD11b⁺/CD33⁺; CD34⁺/Lin⁻/DR⁻/CD11b⁺/CD15⁺ and CD14⁺/HLA-DR⁻/low; CD34⁺/Lin⁻/DR⁻/CD11b⁺/CD15⁺ and Lin⁻/HLA-DR⁻ /low/CD11b⁺/CD33⁺; or CD14⁺/HLA-DR⁻/low and Lin⁻/HLA-DR⁻/low/CD11b⁺/CD33⁺.

In certain embodiments, the cells whose levels are measured in step (i) of the method of the invention are γδ T-cells and any three cell types of the MDSCs listed, i.e., CD11b⁺/CD14⁻, CD11b⁺/CD14⁻/CD15⁺ and CD11b⁺/CD14⁺/CD15⁺; CD11b⁺/CD14⁻, CD11b⁺/CD14⁻/CD15⁺ and Lin⁻/DR⁻; CD11b⁺/CD14⁻, CD11b⁺/CD14⁻/CD15⁺ and Lin⁻/DR⁻ /CD33⁺; CD11b⁺/CD14⁻, CD11b⁺/CD14⁻/CD15⁺ and CD34⁺/CD33⁺/CD13⁺; CD11b⁺/CD14⁻, CD11b⁺/CD14⁻/CD15⁺ and ARG⁺/CD14⁺; CD11b⁺/CD14⁻, CD11b⁺/CD14⁻/CD15⁺ and CD34⁺/Lin⁻/DR⁻/CD11b⁺/CD15⁺; CD11b⁺/CD14⁻, CD11b⁺/CD14⁻/CD15⁺ and CD14⁺/HLA-DR⁻/low; CD11b⁺/CD14⁻, CD11b⁺/CD14⁻/CD15⁺ and Lin⁻/HLA-DR⁻/low/CD11b⁺/CD33⁺; CD11b⁺/CD14⁻, CD11b⁺/CD14⁺/CD15⁺ and Lin⁻ /DR⁻; CD11b⁺/CD14⁻, CD11b⁺/CD14⁺/CD15⁺ and Lin⁻/DR⁻/CD33⁺; CD11b⁺/CD14⁻, CD11b⁺/CD14⁺/CD15⁺ and CD34⁺/CD33⁺/CD13⁺; CD11b⁺/CD14⁻, CD11b⁺/CD14⁺/CD15⁺ and ARG⁺/CD14⁺; CD11b⁺/CD14⁻, CD11b⁺/CD14⁺/CD15⁺ and CD34⁺/Lin⁻/DR⁻/CD1 1b⁺/CD15⁺; CD11b⁺/CD14⁻, CD11b⁺/CD14⁺/CD15⁺ and CD14⁺/HLA-DR⁻/low; CD11b⁺/CD14⁻, CD11b⁺/CD14⁺/CD15⁺ and Lin⁻/HLA-DR⁻ /low/CD11b⁺/CD33⁺; CD11b⁺/CD14⁻, Lin⁻/DR⁻ and Lin⁻/DR⁻/CD33⁺; CD11b⁺/CD14⁻, Lin⁻ /DR⁻ and CD34⁺/CD33⁺/CD13⁺; CD11b⁺/CD14⁻, Lin⁻/DR⁻ and ARG⁺/CD14⁺; CD11b⁺/CD14⁻, Lin⁻/DR⁻ and CD34⁺/Lin⁻/DR⁻/CD11b⁺/CD15⁺; CD11b⁺/CD14⁻, Lin⁻/DR⁻ and CD14⁺/HLA-DR⁻/low; CD11b⁺/CD14⁻, Lin⁻/DR⁻ and Lin⁻/HLA-DR⁻ /low/CD11b⁺/CD33⁺; CD11b⁺/CD14⁻, Lin⁻/DR⁻/CD33⁺ and CD34⁺/CD33⁺/CD13⁺; CD11b⁺/CD14⁻, Lin⁻/DR⁻/CD33⁺ and ARG⁺/CD14⁺; CD11b⁺/CD14⁻, Lin⁻/DR⁻/CD33⁺ and CD34⁺/Lin⁻/DR⁻/CD11b⁺/CD15⁺; CD11b⁺/CD14⁻, Lin⁻/DR⁻/CD33⁺ and CD14⁺/HLA-DR⁻ /low; CD11b⁺/CD14⁻, Lin⁻/DR⁻/CD33⁺ and Lin⁻/HLA-DR⁻/low/CD11b⁺/CD33⁺; CD11b⁺/CD14⁻, CD34⁺/CD33⁺/CD13⁺ and ARG⁺/CD14⁺; CD11b⁺/CD14⁻, CD34⁺/CD33⁺/CD13⁺ and CD34⁺/Lin⁻/DR⁻/CD11b⁺/CD15⁺; CD11b⁺/CD14⁻, CD34⁺/CD33⁺/CD13⁺ and CD14⁺/HLA-DR⁻/low; CD11b⁺/CD14⁻, CD34⁺/CD33⁺/CD13⁺ and Lin⁻/HLA-DR⁻/low/CD11b⁺/CD33⁺; CD11b⁺/CD14⁻, ARG⁺/CD14⁺ and CD34⁺/Lin⁻ /DR⁻/CD11b⁺/CD15⁺; CD11b⁺/CD14⁻, ARG⁺/CD14⁺ and CD14⁺/HLA-DR⁻/low; CD11b⁺/CD14⁻, ARG⁺/CD14⁺ and Lin⁻/HLA-DR⁻/low/CD11b⁺/CD33⁺; CD11b⁺/CD14⁻, CD34⁺/Lin⁻/DR⁻/CD11b⁺/CD15⁺ and CD14⁺/HLA-DR⁻/low; CD11b⁺/CD14⁻, CD34⁺/Lin⁻ /DR⁻/CD11b⁺/CD15⁺ and Lin⁻/HLA-DR⁻/low/CD11b⁺/CD33⁺; CD11b⁺/CD14⁻, CD14⁺/HLA-DR⁻/low and Lin⁻/HLA-DR⁻/low/CD11b⁺/CD33⁺; CD11b⁺/CD14⁻/CD15⁺, CD11b⁺/CD14⁺/CD15⁺ and Lin⁻/DR⁻; CD11b⁺/CD14⁻/CD15⁺, CD11b⁺/CD14⁺/CD15⁺ and Lin⁻/DR⁻/CD33⁺; CD11b⁺/CD14⁻/CD15⁺, CD11b⁺/CD14⁺/CD15⁺ and CD34⁺/CD33⁺/CD13⁺; CD11b⁺/CD14⁻/CD15⁺, CD11b⁺/CD14⁺/CD15⁺ and ARG⁺/CD14⁺; CD11b⁺/CD14⁻/CD15⁺, CD11b⁺/CD14⁺/CD15⁺ and CD34⁺/Lin⁻/DR⁻/CD11b⁺/CD15⁺; CD11b⁺/CD14⁻/CD15⁺, CD11b⁺/CD14⁺/CD15⁺ and CD14⁺/HLA-DR⁻/low; CD11b⁺/CD14⁻ /CD15⁺, CD11b⁺/CD14⁺/CD15⁺ and Lin⁻/HLA-DR⁻/low/CD11b⁺/CD33⁺; CD11b⁺/CD14⁻ /CD15⁺, Lin⁻/DR⁻ and Lin⁻/DR⁻/CD33⁺; CD11b⁺/CD14⁻/CD15⁺, Lin⁻/DR⁻ and CD34⁺/CD33⁺/CD13⁺; CD11b⁺/CD14⁻/CD15⁺, Lin⁻/DR⁻ and ARG⁺/CD14⁺; CD11b⁺/CD14⁻/CD15⁺, Lin⁻/DR⁻ and CD34⁺/Lin⁻/DR⁻/CD11b⁺/CD15⁺; CD11b⁺/CD14⁻ /CD15⁺, Lin⁻/DR⁻ and CD14⁺/HLA-DR⁻/low; CD11b⁺/CD14⁻/CD15⁺, Lin⁻/DR⁻ and Lin⁻ /HLA-DR⁻/low/CD11b⁺/CD33⁺; CD11b⁺/CD14⁻/CD15⁺, Lin⁻/DR⁻/CD33⁺ and CD34⁺/CD33⁺/CD13⁺; CD11b⁺/CD14⁻/CD15⁺, Lin⁻/DR⁻/CD33⁺ and ARG⁺/CD14⁺; CD11b⁺/CD14⁻/CD15⁺, Lin⁻/DR⁻/CD33⁺ and CD34⁺/Lin⁻/DR⁻/CD11b⁺/CD15⁺; CD11b⁺/CD14⁻/CD15⁺, Lin⁻/DR⁻/CD33⁺ and CD14⁺/HLA-DR⁻/low; CD11b⁺/CD14⁻ /CD15⁺, Lin⁻/DR⁻/CD33⁺ and Lin⁻/HLA-DR⁻/low/CD11b⁺/CD33⁺; CD11b⁺/CD14⁻/CD15⁺, CD34⁺/CD33⁺/CD13⁺ and ARG⁺/CD14⁺; CD11b⁺/CD14⁻/CD15⁺, CD34⁺/CD33⁺/CD13⁺ and CD34⁺/Lin⁻/DR⁻/CD11b⁺/CD15⁺; CD11b⁺/CD14⁻/CD15⁺, CD34⁺/CD33⁺/CD13⁺ and CD14⁺/HLA-DR⁻/low; CD11b⁺/CD14⁻/CD15⁺, CD34⁺/CD33⁺/CD13⁺ and Lin⁻/HLA-DR⁻ /low/CD11b⁺/CD33⁺; CD11b⁺/CD14⁻/CD15⁺, ARG⁺/CD14⁺ and CD34⁺/Lin⁻/DR⁻ /CD11b⁺/CD15⁺; CD11b⁺/CD14⁻/CD15⁺, ARG⁺/CD14⁺ and CD14⁺/HLA-DR⁻/low; CD11b⁺/CD14⁻/CD15⁺, ARG⁺/CD14⁺ and Lin⁻/HLA-DR⁻/low/CD11b⁺/CD33⁺; CD11b⁺/CD14⁻/CD15⁺, CD34⁺/Lin⁻/DR⁻/CD11b⁺/CD15⁺ and CD14⁺/HLA-DR⁻/low; CD11b⁺/CD14⁻/CD15⁺, CD34⁺/Lin⁻/DR⁻/CD11b⁺/CD15⁺ and Lin⁻/HLA-DR⁻ /low/CD11b⁺/CD33⁺; CD11b⁺/CD14⁻/CD15⁺, CD14⁺/HLA-DR⁻/low and Lin⁻/HLA-DR⁻ /low/CD11b⁺/CD33⁺; CD11b⁺/CD14⁺/CD15⁺, Lin⁻/DR⁻ and Lin⁻/DR⁻/CD33⁺; CD11b⁺/CD14⁺/CD15⁺, Lin⁻/DR⁻ and CD34⁺/CD33⁺/CD13⁺; CD11b⁺/CD14⁺/CD15⁺, Lin⁻ /DR⁻ and ARG⁺/CD14⁺; CD11b⁺/CD14⁺/CD15⁺, Lin⁻/DR⁻ and CD34⁺/Lin⁻/DR⁻ /CD11b⁺/CD15⁺; CD11b⁺/CD14⁺/CD15⁺, Lin⁻/DR⁻ and CD14⁺/HLA-DR⁻/low; CD11b⁺/CD14⁺/CD15⁺, Lin⁻/DR⁻ and Lin⁻/HLA-DR⁻/low/CD11b⁺/CD33⁺; CD11b⁺/CD14⁺/CD15⁺, Lin⁻/DR⁻/CD33⁺ and CD34⁺/CD33⁺/CD13⁺; CD11b⁺/CD14⁺/CD15⁺, Lin⁻/DR⁻/CD33⁺ and ARG⁺/CD14⁺; CD11b⁺/CD14⁺/CD15⁺, Lin⁻ /DR⁻/CD33⁺ and CD34⁺/Lin⁻/DR⁻/CD11b⁺/CD15⁺; CD11b⁺/CD14⁺/CD15⁺, Lin⁻/DR⁻ /CD33⁺ and CD14⁺/HLA-DR⁻/low; CD11b⁺/CD14⁺/CD15⁺, Lin⁻/DR⁻/CD33⁺ and Lin⁻ /HLA-DR⁻/low/CD11b⁺/CD33⁺; CD11b⁺/CD14⁺/CD15⁺, CD34⁺/CD33⁺/CD13⁺ and ARG⁺/CD14⁺; CD11b⁺/CD14⁺/CD15⁺, CD34⁺/CD33⁺/CD13⁺ and CD34⁺/Lin⁻/DR⁻ /CD11b⁺/CD15⁺; CD11b⁺/CD14⁺/CD15⁺, CD34⁺/CD33⁺/CD13⁺ and CD14⁺/HLA-DR⁻ /low; CD11b⁺/CD14⁺/CD15⁺, CD34⁺/CD33⁺/CD13⁺ and Lin⁻/HLA-DR⁻ /low/CD11b⁺/CD33⁺; CD11b⁺/CD14⁺/CD15⁺, ARG⁺/CD14⁺ and CD34⁺/Lin⁻/DR⁻ /CD11b⁺/CD15⁺; CD11b⁺/CD14⁺/CD15⁺, ARG⁺/CD14⁺ and CD14⁺/HLA-DR/low; CD11b⁺/CD14⁺/CD15⁺, ARG⁺/CD14⁺ and Lin⁻/HLA-DR⁻/low/CD11b⁺/CD33⁺; CD11b⁺/CD14⁺/CD15⁺, CD34⁺/Lin⁻/DR⁻/CD11b⁺/CD15⁺ and CD14⁺/HLA-DR/low; CD11b⁺/CD14⁺/CD15⁺, CD34⁺/Lin⁻/DR⁻/CD11b⁺/CD15⁺ and Lin⁻/HLA-DR⁻/low/CD11b⁺/CD33⁺; Lin⁻/DR⁻, Lin⁻/DR⁻/CD33⁺ and CD34⁺/CD33⁺/CD13⁺; Lin⁻/DR⁻, Lin⁻ /DR⁻/CD33⁺ and ARG⁺/CD14⁺; Lin⁻/DR⁻, Lin⁻/DR⁻/CD33⁺ and CD34⁺/Lin⁻/DR⁻ /CD11b⁺/CD15⁺; Lin⁻/DR⁻, Lin⁻/DR⁻/CD33⁺ and CD14⁺/HLA-DR⁻/low; Lin⁻/DR⁻, Lin⁻/DR⁻ /CD33⁺ and Lin⁻/HLA-DR⁻/low/CD11b⁺/CD33⁺; Lin⁻/DR⁻, CD34⁺/CD33⁺/CD13⁺ and ARG⁺/CD14⁺; Lin⁻/DR⁻, CD34⁺/CD33⁺/CD13⁺ and CD34⁺/Lin⁻/DR⁻/CD11b⁺/CD15⁺; Lin⁻ /DR⁻, CD34⁺/CD33⁺/CD13⁺ and CD14⁺/HLA-DR⁻/low; Lin⁻/DR⁻, CD34⁺/CD33⁺/CD13⁺ and Lin⁻/HLA-DR⁻/low/CD11b⁺/CD33⁺; Lin⁻/DR⁻, ARG⁺/CD14⁺ and CD34⁺/Lin⁻/DR⁻ /CD11b⁺/CD15⁺; Lin⁻/DR⁻, ARG⁺/CD14⁺ and CD14⁺/HLA-DR⁻/low; Lin⁻/DR⁻, ARG⁺/CD14⁺ and Lin⁻/HLA-DR⁻/low/CD11b⁺/CD33⁺; Lin⁻/DR⁻, CD34⁺/Lin⁻/DR⁻ /CD11b⁺/CD15⁺ and CD14⁺/HLA-DR⁻/low; Lin⁻/DR⁻, CD34⁺/Lin⁻/DR⁻/CD11b⁺/CD15⁺ and Lin⁻/HLA-DR⁻/low/CD11b⁺/CD33⁺; Lin⁻/DR⁻, CD14⁺/HLA-DR⁻/low and Lin⁻/HLA-DR⁻/low/CD11b⁺/CD33⁺; Lin/DR⁻/CD33⁺, CD34⁺/CD33⁺/CD13⁺ and ARG⁺/CD14⁺; Lin⁻ /DR⁻/CD33⁺, CD34⁺/CD33⁺/CD13⁺ and CD34⁺/Lin⁻/DR⁻/CD11b⁺/CD15⁺; Lin⁻/DR⁻ /CD33⁺, CD34⁺/CD33⁺/CD13⁺ and CD14⁺/HLA-DR⁻/low; Lin⁻/DR⁻/CD33⁺, CD34⁺/CD33⁺/CD13⁺ and Lin⁻/HLA-DR⁻/low/CD11b⁺/CD33⁺; Lin⁻/DR⁻/CD33⁺, ARG⁺/CD14⁺ and CD34⁺/Lin⁻/DR⁻/CD11b⁺/CD15⁺; Lin⁻/DR⁻/CD33⁺, ARG⁺/CD14⁺ and CD14⁺/HLA-DR⁻/low; Lin/DR⁻/CD33⁺, ARG⁺/CD14⁺ and Lin⁻/HLA-DR⁻ /low/CD 1 1b⁺/CD33⁺; Lin⁻/DR⁻/CD33⁺, CD34⁺/Lin⁻/DR⁻/CD11b⁺/CD15⁺ and CD14⁺/HLA-DR⁻/low; Lin⁻/DR⁻/CD33⁺, CD34⁺/Lin⁻/DR⁻/CD11b⁺/CD15⁺ and Lin⁻/HLA-DR⁻ /low/CD11b⁺/CD33⁺; CD34⁺/CD33⁺/CD13⁺, ARG⁺/CD14⁺ and CD34⁺/Lin⁻/DR⁻ /CD11b⁺/CD15⁺; CD34⁺/CD33⁺/CD13⁺, ARG⁺/CD14⁺ and CD14⁺/HLA-DR⁻/low; CD34⁺/CD33⁺/CD13⁺, ARG⁺/CD14⁺ and Lin⁻/HLA-DR⁻/low/CD11b⁺/CD33⁺; ARG⁺/CD14⁺, CD34⁺/Lin⁻/DR⁻/CD11b⁺/CD15⁺ and CD14⁺/HLA-DR⁻/low; ARG⁺/CD14⁺, CD34⁺/Lin⁻/DR⁻/CD11b⁺/CD15⁺ and Lin⁻/HLA-DR⁻/low/CD11b⁺/CD33⁺; or CD34⁺/Lin⁻ /DR⁻/CD11b⁺/CD15⁺, CD14⁺/HLA-DR⁻/low and Lin⁻/HLA-DR⁻/low/CD11b⁺/CD33⁺.

In certain embodiments, the cells whose levels are measured in step (i) of the method of the invention are γδ T-cells and any four cell types of the MDSCs listed, i.e., CD11b⁺/CD14⁻, CD11b⁺/CD14⁻/CD15⁺, CD11b⁺/CD14⁺/CD15⁺ and Lin⁻/DR⁻; CD11b⁺/CD14⁻, CD11b⁺/CD14⁻/CD15⁺, CD11b⁺/CD14⁺/CD15⁺ and Lin⁻/DR⁻/CD33⁺; CD11b⁺/CD14⁻, CD11b⁺/CD14⁻/CD15⁺, CD11b⁺/CD14⁺/CD15⁺ and CD34⁺/CD33⁺/CD13⁺; CD11b⁺/CD14⁻, CD11b⁺/CD14⁻/CD15⁺, CD11b⁺/CD14⁺/CD15⁺ and ARG⁺/CD14⁺; CD11b⁺/CD14⁻, CD11b⁺/CD14⁻/CD15⁺, CD11b⁺/CD14⁺/CD15⁺ and CD34⁺/Lin⁻/DR⁻/CD11b⁺/CD15⁺; CD11b⁺/CD14⁻, CD11b⁺/CD14⁻/CD15⁺, CD11b⁺/CD14⁺/CD15⁺ and CD14⁺/HLA-DR/low; CD11b⁺/CD14⁻, CD11b⁺/CD14⁻ /CD15⁺, CD11b⁺/CD14⁺/CD15⁺ and Lin⁻/HLA-DR⁻/low/CD11b⁺/CD33⁺; CD11b⁺/CD14⁻, CD11b⁺/CD14⁺/CD15⁺, Lin⁻/DR⁻ and Lin⁻/DR⁻/CD33⁺; CD11b⁺/CD14⁻, CD11b⁺/CD14⁺/CD15⁺, Lin⁻/DR⁻ and CD34⁺/CD33⁺/CD13⁺; CD11b⁺/CD14⁻, CD11b⁺/CD14⁺/CD15⁺, Lin⁻/DR⁻ and ARG⁺/CD14⁺; CD11b⁺/CD14⁻, CD11b⁺/CD14⁺/CD15⁺, Lin⁻/DR⁻ and CD34⁺/Lin⁻/DR⁻/CD11b⁺/CD15⁺; CD11b⁺/CD14⁻, CD11b⁺/CD14⁺/CD15⁺, Lin⁻/DR⁻ and CD14⁺/HLA-DR⁻/low; CD11b⁺/CD14⁻, CD11b⁺/CD14⁺/CD15⁺, Lin⁻/DR⁻ and Lin⁻/HLA-DR⁻/low/CD11b⁺/CD33⁺; CD11b⁺/CD14⁻, Lin⁻/DR⁻, Lin⁻/DR⁻/CD33⁺ and CD34⁺/CD33⁺/CD13⁺; CD11b⁺/CD14⁻, Lin⁻/DR⁻, Lin⁻/DR⁻ /CD33⁺ and ARG⁺/CD14⁺; CD11b⁺/CD14⁻, Lin⁻/DR⁻, Lin⁻/DR⁻/CD33⁺ and CD34⁺/Lin⁻ /DR⁻/CD11b⁺/CD15⁺; CD11b⁺/CD14⁻, Lin⁻/DR⁻, Lin⁻/DR⁻/CD33⁺ and CD14⁺/HLA-DR⁻ /low; CD11b⁺/CD14⁻, Lin⁻/DR⁻, Lin⁻/DR⁻/CD33⁺ and Lin⁻/HLA-DR⁻/low/CD11b⁺/CD33⁺; CD11b⁺/CD14⁻, Lin⁻/DR⁻/CD33⁺, CD34⁺/CD33⁺/CD13⁺ and ARG⁺/CD14⁺; CD11b⁺/CD14⁻, Lin⁻/DR⁻/CD33⁺, CD34⁺/CD33⁺/CD13⁺ and CD34⁺/Lin⁻/DR⁻ /CD11b⁺/CD15⁺; CD11b⁺/CD14⁻, Lin⁻/DR⁻/CD33⁺, CD34⁺/CD33⁺/CD13⁺ and CD14⁺/HLA-DR⁻/low; CD11b⁺/CD14⁻, Lin⁻/DR⁻/CD33⁺, CD34⁺/CD33⁺/CD13⁺ and Lin⁻ /HLA-DR⁻/low/CD11b⁺/CD33⁺; CD11b⁺/CD14⁻, CD34⁺/CD33⁺/CD13⁺, ARG⁺/CD14⁺ and CD34⁺/Lin⁻/DR⁻/CD11b⁺/CD15⁺; CD11b⁺/CD14⁻, CD34⁺/CD33⁺/CD13⁺, ARG⁺/CD14⁺ and CD14⁺/HLA-DR⁻/low; CD11b⁺/CD14⁻, CD34⁺/CD33⁺/CD13⁺, ARG⁺/CD14⁺ and Lin⁻ /HLA-DR⁻/low/CD11b⁺/CD33⁺; CD11b⁺/CD14⁻, ARG⁺/CD14⁺, CD34⁺/Lin⁻/DR⁻ /CD11b⁺/CD15⁺ and CD14⁺/HLA-DR⁻/low; CD11b⁺/CD14⁻, ARG⁺/CD14⁺, CD34⁺/Lin⁻ /DR⁻/CD11b⁺/CD15⁺ and Lin⁻/HLA-DR⁻/low/CD11b⁺/CD33⁺; CD11b⁺/CD14⁻, CD34⁺/Lin⁻ /DR⁻/CD11b⁺/CD15⁺, CD14⁺/HLA-DR⁻/low and Lin⁻/HLA-DR⁻/low/CD11b⁺/CD33⁺; CD11b⁺/CD14⁻/CD15⁺, CD11b⁺/CD14⁺/CD15⁺, Lin⁻/DR⁻ and Lin⁻/DR⁻/CD33⁺; CD11b⁺/CD14⁻/CD15⁺, CD11b⁺/CD14⁺/CD15⁺, Lin⁻/DR⁻ and CD34⁺/CD33⁺/CD13⁺; CD11b⁺/CD14⁻/CD15⁺, CD11b⁺/CD14⁺/CD15⁺, Lin⁻/DR⁻ and ARG⁺/CD14⁺; CD11b⁺/CD14⁻/CD15⁺, CD11b⁺/CD14⁺/CD15⁺, Lin⁻/DR⁻ and CD34⁺/Lin⁻/DR⁻ /CD11b⁺/CD15⁺; CD11b⁺/CD14⁻/CD15⁺, CD11b⁺/CD14⁺/CD15⁺, Lin⁻/DR⁻ and CD14⁺/HLA-DR⁻/low; CD11b⁺/CD14⁻/CD15⁺, CD11b⁺/CD14⁺/CD15⁺, Lin⁻/DR⁻ and Lin⁻ /HLA-DR⁻/low/CD11b⁺/CD33⁺; CD11b⁺/CD14⁻/CD15⁺, Lin⁻/DR⁻, Lin⁻/DR⁻/CD33⁺ and CD34⁺/CD33⁺/CD13⁺; CD11b⁺/CD14⁻/CD15⁺, Lin⁻/DR⁻, Lin⁻/DR⁻/CD33⁺ and ARG⁺/CD14⁺; CD11b⁺/CD14⁻/CD15⁺, Lin⁻/DR⁻, Lin⁻/DR⁻/CD33⁺ and CD34⁺/Lin⁻/DR⁻ /CD11b⁺/CD15⁺; CD11b⁺/CD14⁻/CD15⁺, Lin⁻/DR⁻, Lin⁻/DR⁻/CD33⁺ and CD14⁺/HLA-DR⁻ /low; CD11b⁺/CD14⁻/CD15⁺, Lin⁻/DR⁻, Lin⁻/DR⁻/CD33⁺ and Lin⁻/HLA-DR⁻ /low/CD11b⁺/CD33⁺; CD11b⁺/CD14⁻/CD15⁺, Lin⁻/DR⁻/CD33⁺, CD34⁺/CD33⁺/CD13⁺ and ARG⁺/CD14⁺; CD11b⁺/CD14⁻/CD15⁺, Lin/DR⁻/CD33⁺, CD34⁺/CD33⁺/CD13⁺ and CD34⁺/Lin⁻/DR⁻/CD11b⁺/CD15⁺; CD11b⁺/CD14⁻/CD15⁺, Lin/DR⁻/CD33⁺, CD34⁺/CD33⁺/CD13⁺ and CD14⁺/HLA-DR⁻/low; CD11b⁺/CD14⁻/CD15⁺, Lin⁻/DR⁻ /CD33⁺, CD34⁺/CD33⁺/CD13⁺ and Lin⁻/HLA-DR⁻/low/CD11b⁺/CD33⁺; CD11b⁺/CD14⁻ /CD15⁺, CD34⁺/CD33⁺/CD13⁺, ARG⁺/CD14⁺ and CD34⁺/Lin⁻/DR⁻/CD11b⁺/CD15⁺; CD11b⁺/CD14⁻/CD15⁺, CD34⁺/CD33⁺/CD13⁺, ARG⁺/CD14⁺ and CD14⁺/HLA-DR⁻/low; CD11b⁺/CD14⁻/CD15⁺, CD34⁺/CD33⁺/CD13⁺, ARG⁺/CD14⁺ and Lin⁻/HLA-DR⁻ /low/CD11b⁺/CD33⁺; CD11b⁺/CD14⁻/CD15⁺, ARG⁺/CD14⁺, CD34⁺/Lin⁻/DR⁻ /CD11b⁺/CD15⁺ and CD14⁺/HLA-DR⁻/low; CD11b⁺/CD14⁻/CD15⁺, ARG⁺/CD14⁺, CD34⁺/Lin⁻/DR⁻/CD11b⁺/CD15⁺ and Lin⁻/HLA-DR⁻/low/CD11b⁺/CD33⁺; CD11b⁺/CD14⁻ /CD15⁺, CD34⁺/Lin⁻/DR⁻/CD11b⁺/CD15⁺, CD14⁺/HLA-DR⁻/low and Lin⁻/HLA-DR⁻ /low/CD11b⁺/CD33⁺; CD11b⁺/CD14⁺/CD15⁺, Lin⁻/DR⁻, Lin⁻/DR⁻/CD33⁺ and CD34⁺/CD33⁺/CD13⁺; CD11b⁺/CD14⁺/CD15⁺, Lin⁻/DR⁻, Lin⁻/DR⁻/CD33⁺ and ARG⁺/CD14⁺; CD11b⁺/CD14⁺/CD15⁺, Lin⁻/DR⁻, Lin⁻/DR⁻/CD33⁺ and CD34⁺/Lin⁻/DR⁻ /CD11b⁺/CD15⁺; CD11b⁺/CD14⁺/CD15⁺, Lin⁻/DR⁻, Lin⁻/DR⁻/CD33⁺ and CD14⁺/HLA-DR⁻ /low; CD11b⁺/CD14⁺/CD15⁺, Lin⁻/DR⁻, Lin⁻/DR⁻/CD33⁺ and Lin⁻/HLA-DR⁻ /low/CD11b⁺/CD33⁺; CD11b⁺/CD14⁺/CD15⁺, Lin⁻/DR⁻/CD33⁺, CD34⁺/CD33⁺/CD13⁺ and ARG⁺/CD14⁺; CD11b⁺/CD14⁺/CD15⁺, Lin⁻/DR⁻/CD33⁺, CD34⁺/CD33⁺/CD13⁺ and CD34⁺/Lin⁻/DR⁻/CD11b⁺/CD15⁺; CD11b⁺/CD14⁺/CD15⁺, Lin⁻/DR⁻/CD33⁺, CD34⁺/CD33⁺/CD13⁺ and CD14⁺/HLA-DR⁻/low; CD11b⁺/CD14⁺/CD15⁺, Lin⁻/DR⁻ /CD33⁺, CD34⁺/CD33⁺/CD13⁺ and Lin⁻/HLA-DR⁻/low/CD11b⁺/CD33⁺; CD11b⁺/CD14⁺/CD15⁺, CD34⁺/CD33⁺/CD13⁺, ARG⁺/CD14⁺ and CD34⁺/Lin⁻/DR⁻ /CD11b⁺/CD15⁺; CD11b⁺/CD14⁺/CD15⁺, CD34⁺/CD33⁺/CD13⁺, ARG⁺/CD14⁺ and CD14⁺/HLA-DR⁻/low; CD11b⁺/CD14⁺/CD15⁺, CD34⁺/CD33⁺/CD13⁺, ARG⁺/CD14⁺ and Lin⁻/HLA-DR⁻/low/CD11b⁺/CD33⁺; CD11b⁺/CD14⁺/CD15⁺, ARG⁺/CD14⁺, CD34⁺/Lin⁻ /DR⁻/CD11b⁺/CD15⁺ and CD14⁺/HLA-DR/low; CD11b⁺/CD14⁺/CD15⁺, ARG⁺/CD14⁺, CD34⁺/Lin⁻/DR⁻/CD1 1b⁺/CD15⁺ and Lin⁻/HLA-DR⁻/low/CD11b⁺/CD33⁺; CD11b⁺/CD14⁺/CD15⁺, CD34⁺/Lin⁻/DK/CD11b⁺/CD15⁺, CD14⁺/HLA-DR⁻/low and Lin⁻ /HLA-DR⁻/low/CD11b⁺/CD33⁺; Lin⁻/DR⁻, Lin⁻/DR⁻/CD33⁺, CD34⁺/CD33⁺/CD13⁺ and ARG⁺/CD14⁺; Lin⁻/DR⁻, Lin⁻/DR⁻/CD33⁺, CD34⁺/CD33⁺/CD13⁺ and CD34⁺/Lin⁻/DR⁻ /CD11b⁺/CD15⁺; Lin⁻/DR⁻, Lin⁻/DR⁻/CD33⁺, CD34⁺/CD33⁺/CD13⁺ and CD14⁺/HLA-DR⁻ /low; Lin⁻/DR⁻, Lin⁻/DR⁻/CD33⁺, CD34⁺/CD33⁺/CD13⁺ and Lin⁻/HLA-DR⁻ /low/CD 1 1b⁺/CD33⁺; Lin⁻/DR⁻, CD34⁺/CD33⁺/CD13⁺, ARG⁺/CD14⁺ and CD34⁺/Lin⁻/DR⁻ /CD11b⁺/CD15⁺; Lin⁻/DR⁻, CD34⁺/CD33⁺/CD13⁺, ARG⁺/CD14⁺ and CD14⁺/HLA-DR⁻ /low; Lin⁻/DR⁻, CD34⁺/CD33⁺/CD13⁺, ARG⁺/CD14⁺ and Lin⁻/HLA-DR⁻ /low/CD11b⁺/CD33⁺; Lin⁻/DR⁻, ARG⁺/CD14⁺, CD34⁺/Lin⁻/DR⁻/CD11b⁺/CD15⁺ and CD14⁺/HLA-DR⁻/low; Lin⁻/DR⁻, ARG⁺/CD14⁺, CD34⁺/Lin⁻/DR⁻/CD11b⁺/CD15⁺ and Lin⁻ /HLA-DR⁻/low/CD11b⁺/CD33⁺; Lin⁻/DR⁻/CD33⁺, CD34⁺/CD33⁺/CD13⁺, ARG⁺/CD14⁺ and CD34⁺/Lin⁻/DR⁻/CD11b⁺/CD15⁺; Lin⁻/DR⁻/CD33⁺, CD34⁺/CD33⁺/CD13⁺, ARG⁺/CD14⁺ and CD14⁺/HLA-DR⁻/low; Lin⁻/DR⁻/CD33⁺, CD34⁺/CD33⁺/CD13⁺, ARG⁺/CD14⁺ and Lin⁻/HLA-DR⁻/low/CD11b⁺/CD33⁺; Lin⁻/DR⁻/CD33⁺, ARG⁺/CD14⁺, CD34⁺/Lin⁻/DR⁻/CD1 1b⁺/CD15⁺ and CD14⁺/HLA-DR⁻/low; Lin⁻/DR⁻/CD33⁺, ARG⁺/CD14⁺, CD34⁺/Lin⁻/DR⁻/CD11b⁺/CD15⁺ and Lin⁻/HLA-DR⁻/low/CD11b⁺/CD33⁺; Lin⁻/DR⁻/CD33⁺, CD34⁺/Lin⁻/DR⁻/CD11b⁺/CD15⁺, CD14⁺/HLA-DR⁻/low and Lin⁻/HLA-DR⁻/low/CD11b⁺/CD33⁺; CD34⁺/CD33⁺/CD13⁺, ARG⁺/CD14⁺, CD34⁺/Lin⁻/DR⁻ /CD11b⁺/CD15⁺ and CD14⁺/HLA-DR⁻/low; CD34⁺/CD33⁺/CD13⁺, ARG⁺/CD14⁺, CD34⁺/Lin⁻/DR⁻/CD1 1b⁺/CD15⁺ and Lin⁻/HLA-DR⁻/low/CD11b⁺/CD33⁺; CD34⁺/CD33⁺/CD13⁺, CD34⁺/Lin⁻/DR⁻/CD11b⁺/CD15⁺, CD14⁺/HLA-DR⁻/low and Lin⁻ /HLA-DR⁻/low/CD11b⁺/CD33⁺; or ARG⁺/CD14⁺, CD34⁺/Lin⁻/DK/CD11b⁺/CD15⁺, CD14⁺/HLA-DR⁻/low and Lin-/HLA-DR⁻/low/CD11b⁺/CD33⁺.

In certain embodiments, the cells whose levels are measured in step (i) of the method of the invention are γδ T-cells and any five cell types of the MDSCs listed, i.e., CD11b⁺/CD14⁻, CD11b⁺/CD14⁻/CD15⁺, CD11b⁺/CD14⁺/CD15⁺, Lin⁻/DR⁻ and Lin⁻/DR⁻ /CD33⁺; CD11b⁺/CD14⁻, CD11b⁺/CD14⁻/CD15⁺, CD11b⁺/CD14⁺/CD15⁺, Lin⁻/DR⁻ and CD34⁺/CD33⁺/CD13⁺; CD11b⁺/CD14⁻, CD11b⁺/CD14⁻/CD15⁺, CD11b⁺/CD14⁺/CD15⁺, Lin⁻/DR⁻ and ARG⁺/CD14⁺; CD11b⁺/CD14⁻, CD11b⁺/CD14⁻/CD15⁺, CD11b⁺/CD14⁺/CD15⁺, Lin⁻/DR⁻ and CD34⁺/Lin⁻/DR⁻/CD11b⁺/CD15⁺; CD11b⁺/CD14⁻, CD11b⁺/CD14⁻/CD15⁺, CD11b⁺/CD14⁺/CD15⁺, Lin⁻/DR⁻ and CD14⁺/HLA-DR⁻/low; CD11b⁺/CD14⁻, CD11b⁺/CD14⁻/CD15⁺, CD11b⁺/CD14⁺/CD15⁺, Lin⁻/DR⁻ and Lin⁻/HLA-DR⁻/low/CD11b⁺/CD33⁺; CD11b⁺/CD14⁻, CD11b⁺/CD14⁺/CD15⁺, Lin⁻/DR⁻, Lin⁻/DR⁻ /CD33⁺ and CD34⁺/CD33⁺/CD13⁺; CD11b⁺/CD14⁻, CD11b⁺/CD14⁺/CD15⁺, Lin⁻/DR⁻, Lin⁻ /DR⁻/CD33⁺ and ARG⁺/CD14⁺; CD11b⁺/CD14⁻, CD11b⁺/CD14⁺/CD15⁺, Lin⁻/DR⁻, Lin⁻ /DR⁻/CD33⁺ and CD34⁺/Lin⁻/DR⁻/CD11b⁺/CD15⁺; CD11b⁺/CD14⁻, CD11b⁺/CD14⁺/CD15⁺, Lin⁻/DR⁻, Lin⁻/DR⁻/CD33⁺ and CD14⁺/HLA-DR⁻/low; CD11b⁺/CD14⁻, CD11b⁺/CD14⁺/CD15⁺, Lin⁻/DR⁻, Lin⁻/DR⁻/CD33⁺ and Lin⁻/HLA-DR⁻ /low/CD11b⁺/CD33⁺; CD11b⁺/CD14⁻, Lin⁻/DR⁻, Lin⁻/DR⁻/CD33⁺, CD34⁺/CD33⁺/CD13⁺ and ARG⁺/CD14⁺; CD11b⁺/CD14⁻, Lin⁻/DR⁻, Lin⁻/DR⁻/CD33⁺, CD34⁺/CD33⁺/CD13⁺ and CD34⁺/Lin⁻/DR⁻/CD11b⁺/CD15⁺; CD11b⁺/CD14⁻, Lin⁻/DR⁻, Lin⁻/DR⁻/CD33⁺, CD34⁺/CD33⁺/CD13⁺ and CD14⁺/HLA-DR⁻/low; CD11b⁺/CD14⁻, Lin⁻/DR⁻, Lin⁻/DR⁻ /CD33⁺, CD34⁺/CD33⁺/CD13⁺ and Lin⁻/HLA-DR⁻/low/CD11b⁺/CD33⁺; CD11b⁺/CD14⁻, Lin⁻/DR⁻/CD33⁺, CD34⁺/CD33⁺/CD13⁺, ARG⁺/CD14⁺ and CD34⁺/Lin⁻/DR⁻ /CD11b⁺/CD15⁺; CD11b⁺/CD14⁻, Lin⁻/DR⁻/CD33⁺, CD34⁺/CD33⁺/CD13⁺, ARG⁺/CD14⁺ and CD14⁺/HLA-DR⁻/low; CD11b⁺/CD14⁻, Lin⁻/DR⁻/CD33⁺, CD34⁺/CD33⁺/CD13⁺, ARG⁺/CD14⁺ and Lin⁻/HLA-DK/low/CD11b⁺/CD33⁺; CD11b⁺/CD14⁻, CD34⁺/CD33⁺/CD13⁺, ARG⁺/CD14⁺, CD34⁺/Lin⁻/DR⁻/CD11b⁺/CD15⁺ and CD14⁺/HLA-DR⁻/low; CD11b⁺/CD14⁻, CD34⁺/CD33⁺/CD13⁺, ARG⁺/CD14⁺, CD34⁺/Lin⁻/DR⁻ /CD11b⁺/CD15⁺ and Lin⁻/HLA-DR/low/CD11b⁺/CD33⁺; CD11b⁺/CD14⁻, ARG⁺/CD14⁺, CD34⁺/Lin⁻/DR⁻/CD11b⁺/CD15⁺, CD14⁺/HLA-DR⁻/low and Lin⁻/HLA-DR⁻ /low/CD11b⁺/CD33⁺; CD11b⁺/CD14⁻/CD15⁺, CD11b⁺/CD14⁺/CD15⁺, Lin⁻/DR⁻, Lin⁻/DR⁻ /CD33⁺ and CD34⁺/CD33⁺/CD13⁺; CD11b⁺/CD14⁻/CD15⁺, CD11b⁺/CD14⁺/CD15⁺, Lin⁻ /DR⁻, Lin⁻/DR⁻/CD33⁺ and ARG⁺/CD14⁺; CD11b⁺/CD14⁻/CD15⁺, CD11b⁺/CD14⁺/CD15⁺, Lin⁻/DR⁻, Lin⁻/DR⁻/CD33⁺ and CD34⁺/Lin⁻/DR⁻/CD11b⁺/CD15⁺; CD11b⁺/CD14⁻/CD15⁺, CD11b⁺/CD14⁺/CD15⁺, Lin⁻/DR⁻, Lin⁻/DR⁻/CD33⁺ and CD14⁺/HLA-DR⁻/low; CD11b⁺/CD14⁻/CD15⁺, CD11b⁺/CD14⁺/CD15⁺, Lin⁻/DR⁻, Lin⁻/DR⁻/CD33⁺ and Lin⁻/HLA-DR⁻/low/CD11b⁺/CD33⁺; CD11b⁺/CD14⁻/CD15⁺, Lin⁻/DR⁻, Lin⁻/DR⁻/CD33⁺, CD34⁺/CD33⁺/CD13⁺ and ARG⁺/CD14⁺; CD11b⁺/CD14⁻/CD15⁺, Lin⁻/DR⁻, Lin⁻/DR⁻ /CD33⁺, CD34⁺/CD33⁺/CD13⁺ and CD34⁺/Lin⁻/DR⁻/CD11b⁺/CD15⁺; CD11b⁺/CD14⁻ /CD15⁺, Lin⁻/DR⁻, Lin⁻/DR⁻/CD33⁺, CD34⁺/CD33⁺/CD13⁺ and CD14⁺/HLA-DR⁻/low; CD11b⁺/CD14⁻/CD15⁺, Lin⁻/DR⁻, Lin⁻/DR⁻/CD33⁺, CD34⁺/CD33⁺/CD13⁺ and Lin⁻/HLA-DR⁻/low/CD11b⁺/CD33⁺; CD11b⁺/CD14⁻/CD15⁺, Lin⁻/DR⁻/CD33⁺, CD34⁺/CD33⁺/CD13⁺, ARG⁺/CD14⁺ and CD34⁺/Lin⁻/DR⁻/CD11b⁺/CD15⁺; CD11b⁺/CD14⁻/CD15⁺, Lin⁻/DR⁻ /CD33⁺, CD34⁺/CD33⁺/CD13⁺, ARG⁺/CD14⁺ and CD14⁺/HLA-DR⁻/low; CD11b⁺/CD14⁻ /CD15⁺, Lin⁻/DR⁻/CD33⁺, CD34⁺/CD33⁺/CD13⁺, ARG⁺/CD14⁺ and Lin⁻/HLA-DR⁻ /low/CD11b⁺/CD33⁺; CD11b⁺/CD14⁻/CD15⁺, CD34⁺/CD33⁺/CD13⁺, ARG⁺/CD14⁺, CD34⁺/Lin⁻/DR⁻/CD11b⁺/CD15⁺ and CD14⁺/HLA-DR⁻/low; CD11b⁺/CD14⁻/CD15⁺, CD34⁺/CD33⁺/CD13⁺, ARG⁺/CD14⁺, CD34⁺/Lin⁻/DR⁻/CD11b⁺/CD15⁺ and Lin⁻/HLA-DR⁻ /low/CD11b⁺/CD33⁺; CD11b⁺/CD14⁻/CD15⁺, ARG⁺/CD14⁺, CD34⁺/Lin⁻/DR⁻ /CD11b⁺/CD15⁺, CD14⁺/HLA-DR⁻/low and Lin⁻/HLA-DR⁻/low/CD11b⁺/CD33⁺; CD11b⁺/CD14⁺/CD15⁺, Lin⁻/DR⁻, Lin⁻/DR⁻/CD33⁺, CD34⁺/CD33⁺/CD13⁺ and ARG⁺/CD14⁺; CD11b⁺/CD14⁺/CD15⁺, Lin⁻/DR⁻, Lin⁻/DR⁻/CD33⁺, CD34⁺/CD33⁺/CD13⁺ and CD34⁺/Lin⁻/DR⁻/CD11b⁺/CD15⁺; CD11b⁺/CD14⁺/CD15⁺, Lin⁻/DR⁻, Lin⁻/DR⁻/CD33⁺, CD34⁺/CD33⁺/CD13⁺ and CD14⁺/HLA-DR⁻/low; CD11b⁺/CD14⁺/CD15⁺, Lin⁻/DR⁻, Lin⁻ /DR⁻/CD33⁺, CD34⁺/CD33⁺/CD13⁺ and Lin⁻/HLA-DK/low/CD11b⁺/CD33⁺; CD11b⁺/CD14⁺/CD15⁺, Lin⁻/DR⁻/CD33⁺, CD34⁺/CD33⁺/CD13⁺, ARG⁺/CD14⁺ and CD34⁺/Lin⁻/DR⁻/CD11b⁺/CD15⁺; CD11b⁺/CD14⁺/CD15⁺, Lin⁻/DR⁻/CD33⁺, CD34⁺/CD33⁺/CD13⁺, ARG⁺/CD14⁺ and CD14⁺/HLA-DR⁻/low; CD11b⁺/CD14⁺/CD15⁺, Lin⁻/DR⁻/CD33⁺, CD34⁺/CD33⁺/CD13⁺, ARG⁺/CD14⁺ and Lin⁻/HLA-DR⁻ /low/CD11b⁺/CD33⁺; CD11b⁺/CD14⁺/CD15⁺, CD34⁺/CD33⁺/CD13⁺, ARG⁺/CD14⁺, CD34⁺/Lin⁻/DR⁻/CD11b⁺/CD15⁺ and CD14⁺/HLA-DR⁻/low; CD11b⁺/CD14⁺/CD15⁺, CD34⁺/CD33⁺/CD13⁺, ARG⁺/CD14⁺, CD34⁺/Lin⁻/DR⁻/CD11b⁺/CD15⁺ and Lin⁻/HLA-DR⁻ /low/CD11b⁺/CD33⁺; CD11b⁺/CD14⁺/CD15⁺, ARG⁺/CD14⁺, CD34⁺/Lin⁻/DR⁻ /CD11b⁺/CD15⁺, CD14⁺/HLA-DR⁻/low and Lin⁻/HLA-DR/low/CD11b⁺/CD33⁺; Lin⁻/DR⁻, Lin⁻/DR⁻/CD33⁺, CD34⁺/CD33⁺/CD13⁺, ARG⁺/CD14⁺ and CD34⁺/Lin⁻/DR⁻ /CD11b⁺/CD15⁺; Lin⁻/DR⁻, Lin⁻/DR⁻/CD33⁺, CD34⁺/CD33⁺/CD13⁺, ARG⁺/CD14⁺ and CD14⁺/HLA-DR⁻/low; Lin⁻/DR⁻, Lin⁻/DR⁻/CD33⁺, CD34⁺/CD33⁺/CD13⁺, ARG⁺/CD14⁺ and Lin⁻/HLA-DR⁻/low/CD11b⁺/CD33⁺; Lin⁻/DR⁻, CD34⁺/CD33⁺/CD13⁺, ARG⁺/CD14⁺, CD34⁺/Lin⁻/DR⁻/CD11b⁺/CD15⁺ and CD14⁺/HLA-DR⁻/low; Lin⁻/DR⁻, CD34⁺/CD33⁺/CD13⁺, ARG⁺/CD14⁺, CD34⁺/Lin⁻/DR⁻/CD11b⁺/CD15⁺ and Lin⁻/HLA-DR⁻ /low/CD11b⁺/CD33⁺; Lin⁻/DR⁻, ARG⁺/CD14⁺, CD34⁺/Lin⁻/DR⁻/CD11b⁺/CD15⁺, CD14⁺/HLA-DR⁻/low and Lin⁻/HLA-DR⁻/low/CD11b⁺/CD33⁺; Lin⁻/DR⁻/CD33⁺, CD34⁺/CD33⁺/CD13⁺, ARG⁺/CD14⁺, CD34⁺/Lin /DR⁻/CD11b⁺/CD15⁺ and CD14⁺/HLA-DR⁻/low; Lin⁻/DR⁻/CD33⁺, CD34⁺/CD33⁺/CD13⁺, ARG⁺/CD14⁺, CD34⁺/Lin⁻/DR⁻ /CD11b⁺/CD15⁺ and Lin⁻/HLA-DR⁻/low/CD11b⁺/CD33⁺; Lin⁻/DR⁻/CD33⁺, ARG⁺/CD14⁺, CD34⁺/Lin⁻/DR⁻/CD11b⁺/CD15⁺, CD14⁺/HLA-DR⁻/low and Lin⁻/HLA-DR⁻ /low/CD11b⁺/CD33⁺; or CD34⁺/CD33⁺/CD13⁺, ARG⁺/CD14⁺, CD34⁺/Lin⁻/DR⁻ /CD11b⁺/CD15⁺, CD14⁺/HLA-DR⁻/low and Lin⁻/HLA-DR⁻/low/CD11b⁺/CD33⁺.

In certain embodiments, the cells whose levels are measured in step (i) of the method of the invention are γδ T-cells and any six cell types of the MDSCs listed, i.e., CD11b⁺/CD14⁻, CD11b⁺/CD14⁻/CD15⁺, CD11b⁺/CD14⁺/CD15⁺, Lin⁻/DR⁻, Lin⁻/DR⁻ /CD33⁺ and CD34⁺/CD33⁺/CD13⁺; CD11b⁺/CD14⁻, CD11b⁺/CD14⁻/CD15⁺, CD11b⁺/CD14⁺/CD15⁺, Lin⁻/DR⁻, Lin⁻/DR⁻/CD33⁺ and ARG⁺/CD14⁺; CD11b⁺/CD14⁻, CD11b⁺/CD14⁻/CD15⁺, CD11b⁺/CD14⁺/CD15⁺, Lin⁻/DR⁻, Lin⁻/DR⁻/CD33⁺ and CD34⁺/Lin⁻/DR⁻/CD11b⁺/CD15⁺; CD11b⁺/CD14⁻, CD11b⁺/CD14⁻/CD15⁺, CD11b⁺/CD14⁺/CD15⁺, Lin⁻/DR⁻, Lin⁻/DR⁻/CD33⁺ and CD14⁺/HLA-DR⁻/low; CD11b⁺/CD14⁻, CD11b⁺/CD14⁻/CD15⁺, CD11b⁺/CD14⁺/CD15⁺, Lin⁻/DR⁻, Lin⁻/DR⁻ /CD33⁺ and Lin⁻/HLA-DR/low/CD11b⁺/CD33⁺; CD11b⁺/CD14⁻, CD11b⁺/CD14⁺/CD15⁺, Lin⁻/DR⁻, Lin⁻/DR⁻/CD33⁺, CD34⁺/CD33⁺/CD13⁺ and ARG⁺/CD14⁺; CD11b⁺/CD14⁻, CD11b⁺/CD14⁺/CD15⁺, Lin⁻/DR⁻, Lin⁻/DR⁻/CD33⁺, CD34⁺/CD33⁺/CD13⁺ and CD34⁺/Lin⁻ /DR⁻/CD11b⁺/CD15⁺; CD11b⁺/CD14⁻, CD11b⁺/CD14⁺/CD15⁺, Lin⁻/DR⁻, Lin⁻/DR⁻/CD33⁺, CD34⁺/CD33⁺/CD13⁺ and CD14⁺/HLA-DR⁻/low; CD11b⁺/CD14⁻, CD11b⁺/CD14⁺/CD15⁺, Lin⁻/DR⁻, Lin⁻/DR⁻/CD33⁺, CD34⁺/CD33⁺/CD13⁺ and Lin⁻/HLA-DR⁻/low/CD11b⁺/CD33⁺; CD11b⁺/CD14⁻, Lin⁻/DR⁻, Lin⁻/DR⁻/CD33⁺, CD34⁺/CD33⁺/CD13⁺, ARG⁺/CD14⁺ and CD34⁺/Lin⁻/DR⁻/CD11b⁺/CD15⁺; CD11b⁺/CD14⁻, Lin⁻/DR⁻, Lin⁻/DR⁻/CD33⁺, CD34⁺/CD33⁺/CD13⁺, ARG⁺/CD14⁺ and CD14⁺/HLA-DR⁻/low; CD11b⁺/CD14⁻, Lin⁻/DR⁻, Lin⁻/DR⁻/CD33⁺, CD34⁺/CD33⁺/CD13⁺, ARG⁺/CD14⁺ and Lin⁻/HLA-DR⁻ /low/CD11b⁺/CD33⁺; CD11b⁺/CD14⁻, Lin⁻/DR⁻/CD33⁺, CD34⁺/CD33⁺/CD13⁺, ARG⁺/CD14⁺, CD34⁺/Lin⁻/DR⁻/CD11b⁺/CD15⁺ and CD14⁺/HLA-DR⁻/low; CD11b⁺/CD14⁻ , Lin⁻/DR⁻/CD33⁺, CD34⁺/CD33⁺/CD13⁺, ARG⁺/CD14⁺, CD34⁺/Lin⁻/DR⁻/CD11b⁺/CD15⁺ and Lin⁻/HLA-DR⁻/low/CD11b⁺/CD33⁺; or CD11b⁺/CD14⁻, CD34⁺/CD33⁺/CD13⁺, ARG⁺/CD14⁺, CD34⁺/Lin⁻/DR⁻/CD11b⁺/CD15⁺, CD14⁺/HLA-DR⁻/low and Lin⁻/HLA-DR⁻ /low/CD11b⁺/CD33⁺; CD11b⁺/CD14⁻/CD15⁺, CD11b⁺/CD14⁺/CD15⁺, Lin⁻/DR⁻, Lin⁻/DR⁻ /CD33⁺, CD34⁺/CD33⁺/CD13⁺ and ARG⁺/CD14⁺; CD11b⁺/CD14⁻/CD15⁺, CD11b⁺/CD14⁺/CD15⁺, Lin⁻/DR⁻, Lin⁻/DR⁻/CD33⁺, CD34⁺/CD33⁺/CD13⁺ and CD34⁺/Lin⁻ /DR⁻/CD11b⁺/CD15⁺; CD11b⁺/CD14⁻/CD15⁺, CD11b⁺/CD14⁺/CD15⁺, Lin⁻/DR⁻, Lin⁻/DR⁻ /CD33⁺, CD34⁺/CD33⁺/CD13⁺ and CD14⁺/HLA-DR⁻/low; CD11b⁺/CD14⁻/CD15⁺, CD11b⁺/CD14⁺/CD15⁺, Lin⁻/DR⁻, Lin⁻/DR⁻/CD33⁺, CD34⁺/CD33⁺/CD13⁺ and Lin⁻/HLA-DR⁻/low/CD11b⁺/CD33⁺; CD11b⁺/CD14⁻/CD15⁺, Lin⁻/DR⁻, Lin⁻/DR⁻/CD33⁺, CD34⁺/CD33⁺/CD13⁺, ARG⁺/CD14⁺ and CD34⁺/Lin⁻/DR⁻/CD11b⁺/CD15⁺; CD11b⁺/CD14⁻/CD15⁺, Lin⁻/DR⁻, Lin⁻/DR⁻/CD33⁺, CD34⁺/CD33⁺/CD13⁺, ARG⁺/CD14⁺ and CD14⁺/HLA-DR⁻/low; CD11b⁺/CD14⁻/CD15⁺, Lin⁻/DR⁻, Lin⁻/DR⁻/CD33⁺, CD34⁺/CD33⁺/CD13⁺, ARG⁺/CD14⁺ and Lin⁻/HLA-DR⁻/low/CD11b⁺/CD33⁺; CD11b⁺/CD14⁻/CD15⁺, Lin⁻/DR⁻/CD33⁺, CD34⁺/CD33⁺/CD13⁺, ARG⁺/CD14⁺, CD34⁺/Lin⁻/DR⁻/CD11b⁺/CD15⁺ and CD14⁺/HLA-DR⁻/low; CD11b⁺/CD14⁻/CD15⁺, Lin⁻ /DR⁻/CD33⁺, CD34⁺/CD33⁺/CD13⁺, ARG⁺/CD14⁺, CD34⁺/Lin⁻/DR⁻/CD11b⁺/CD15⁺ and Lin⁻/HLA-DR⁻/low/CD11b⁺/CD33⁺; CD11b⁺/CD14⁻/CD15⁺, CD34⁺/CD33⁺/CD13⁺, ARG⁺/CD14⁺, CD34⁺/Lin⁻/DR⁻/CD11b⁺/CD15⁺, CD14⁺/HLA-DR⁻/low and Lin⁻/HLA-DR⁻ /low/CD11b⁺/CD33⁺; CD11b⁺/CD14⁺/CD15⁺, Lin⁻/DR⁻, Lin⁻/DR⁻/CD33⁺, CD34⁺/CD33⁺/CD13⁺, ARG⁺/CD14⁺ and CD34⁺/Lin⁻/DR⁻/CD11b⁺/CD15⁺; CD11b⁺/CD14⁺/CD15⁺, Lin⁻/DR⁻, Lin⁻/DR⁻/CD33⁺, CD34⁺/CD33⁺/CD13⁺, ARG⁺/CD14⁺ and CD14⁺/HLA-DR⁻/low; CD11b⁺/CD14⁺/CD15⁺, Lin⁻/DR⁻, Lin⁻/DR⁻/CD33⁺, CD34⁺/CD33⁺/CD13⁺, ARG⁺/CD14⁺ and Lin⁻/HLA-DK/low/CD11b⁺/CD33⁺; CD11b⁺/CD14⁺/CD15⁺, Lin⁻/DR⁻/CD33⁺, CD34⁺/CD33⁺/CD13⁺, ARG⁺/CD14⁺, CD34⁺/Lin⁻/DR⁻/CD11b⁺/CD15⁺ and CD14⁺/HLA-DR⁻/low; CD11b⁺/CD14⁺/CD15⁺, Lin⁻ /DR⁻/CD33⁺, CD34⁺/CD33⁺/CD13⁺, ARG⁺/CD14⁺, CD34⁺/Lin⁻/DR⁻/CD11b⁺/CD15⁺ and Lin⁻/HLA-DR⁻/low/CD11b⁺/CD33⁺; CD11b⁺/CD14⁺/CD15⁺, CD34⁺/CD33⁺/CD13⁺, ARG⁺/CD14⁺, CD34⁺/Lin⁻/DR⁻/CD11b⁺/CD15⁺, CD14⁺/HLA-DR⁻/low and Lin⁻/HLA-DR⁻ /low/CD11b⁺/CD33⁺; Lin⁻/DR⁻, Lin⁻/DR⁻/CD33⁺, CD34⁺/CD33⁺/CD13⁺, ARG⁺/CD14⁺, CD34⁺/Lin⁻/DR⁻/CD11b⁺/CD15⁺ and CD14⁺/HLA-DR⁻/low; Lin⁻/DR⁻, Lin⁻/DR⁻/CD33⁺, CD34⁺/CD33⁺/CD13⁺, ARG⁺/CD14⁺, CD34⁺/Lin⁻/DR⁻/CD11b⁺/CD15⁺ and Lin⁻/HLA-DR⁻ /low/CD11b⁺/CD33⁺; Lin⁻/DR⁻, CD34⁺/CD33⁺/CD13⁺, ARG⁺/CD14⁺, CD34⁺/Lin⁻/DR⁻ /CD11b⁺/CD15⁺, CD14⁺/HLA-DR⁻/low and Lin⁻/HLA-DR⁻/low/CD11b⁺/CD33⁺; or Lin⁻ /DR⁻/CD33⁺, CD34⁺/CD33⁺/CD13⁺, ARG⁺/CD14⁺, CD34⁺/Lin⁻/DR⁻/CD11b⁺/CD15⁺, CD14⁺/HLA-DR⁻/low and Lin⁻/HLA-DR⁻/low/CD11b⁺/CD33⁺.

In certain embodiments, the cells whose levels are measured in step (i) of the method of the invention are γδ T-cells and any seven cell types of the MDSCs listed, i.e., CD11b⁺/CD14⁻, CD11b⁺/CD14⁻/CD15⁺, CD11b⁺/CD14⁺/CD15⁺, Lin⁻/DR⁻, Lin⁻/DR⁻ /CD33⁺, CD34⁺/CD33⁺/CD13⁺ and ARG⁺/CD14⁺; CD11b⁺/CD14⁻, CD11b⁺/CD14⁻/CD15⁺, CD11b⁺/CD14⁺/CD15⁺, Lin⁻/DR⁻, Lin⁻/DR⁻/CD33⁺, CD34⁺/CD33⁺/CD13⁺ and CD34⁺/Lin⁻ /DR⁻/CD11b⁺/CD15⁺; CD11b⁺/CD14⁻, CD11b⁺/CD14⁻/CD15⁺, CD11b⁺/CD14⁺/CD15⁺, Lin⁻/DR⁻, Lin⁻/DR⁻/CD33⁺, CD34⁺/CD33⁺/CD13⁺ and CD14⁺/HLA-DR⁻/low; CD11b⁺/CD14⁻, CD11b⁺/CD14⁻/CD15⁺, CD11b⁺/CD14⁺/CD15⁺, Lin⁻/DR⁻, Lin⁻/DR⁻ /CD33⁺, CD34⁺/CD33⁺/CD13⁺ and Lin⁻/HLA-DR⁻/low/CD11b⁺/CD33⁺; CD11b⁺/CD14⁻, CD11b⁺/CD14⁺/CD15⁺, Lin⁻/DR⁻, Lin⁻/DR⁻/CD33⁺, CD34⁺/CD33⁺/CD13⁺, ARG⁺/CD14⁺ and CD34⁺/Lin⁻/DR⁻/CD11b⁺/CD15⁺; CD11b⁺/CD14⁻, CD11b⁺/CD14⁺/CD15⁺, Lin⁻/DR⁻, Lin⁻/DR⁻/CD33⁺, CD34⁺/CD33⁺/CD13⁺, ARG⁺/CD14⁺ and CD14⁺/HLA-DR⁻/low; CD11b⁺/CD14⁻, CD11b⁺/CD14⁺/CD15⁺, Lin⁻/DR⁻, Lin⁻/DR⁻/CD33⁺, CD34⁺/CD33⁺/CD13⁺, ARG⁺/CD14⁺ and Lin⁻/HLA-DR⁻/low/CD11b⁺/CD33⁺; CD11b⁺/CD14⁻, Lin⁻/DR⁻, Lin⁻/DR⁻/CD33⁺, CD34⁺/CD33⁺/CD13⁺, ARG⁺/CD14⁺, CD34⁺/Lin⁻/DR⁻/CD11b⁺/CD15⁺ and CD14⁺/HLA-DR⁻/low; CD11b⁺/CD14⁻, Lin⁻/DR⁻, Lin⁻/DR⁻/CD33⁺, CD34⁺/CD33⁺/CD13⁺, ARG⁺/CD14⁺, CD34⁺/Lin⁻/DR⁻/CD11b⁺/CD15⁺ and Lin⁻/HLA-DR⁻/low/CD11b⁺/CD33⁺; CD11b⁺/CD14⁻, Lin⁻/DR⁻/CD33⁺, CD34⁺/CD33⁺/CD13⁺, ARG⁺/CD14⁺, CD34⁺/Lin⁻/DR⁻/CD11b⁺/CD15⁺, CD14⁺/HLA-DR⁻ /low and Lin⁻/HLA-DR⁻/low/CD11b⁺/CD33⁺; CD11b⁺/CD14⁻/CD15⁺, CD11b⁺/CD14⁺/CD15⁺, Lin⁻/DR⁻, Lin⁻/DR⁻/CD33⁺, CD34⁺/CD33⁺/CD13⁺, ARG⁺/CD14⁺ and CD34⁺/Lin⁻/DR⁻/CD11b⁺/CD15⁺; CD11b⁺/CD14⁻/CD15⁺, CD11b⁺/CD14⁺/CD15⁺, Lin⁻ /DR⁻, Lin⁻/DR⁻/CD33⁺, CD34⁺/CD33⁺/CD13⁺, ARG⁺/CD14⁺ and CD14⁺/HLA-DR⁻/low; CD11b⁺/CD14⁻/CD15⁺, CD11b⁺/CD14⁺/CD15⁺, Lin⁻/DR⁻, Lin⁻/DR⁻/CD33⁺, CD34⁺/CD33⁺/CD13⁺, ARG⁺/CD14⁺ and Lin⁻/HLA-DK/low/CD11b⁺/CD33⁺; CD11b⁺/CD14⁻/CD15⁺, Lin⁻/DR⁻, Lin⁻/DR⁻/CD33⁺, CD34⁺/CD33⁺/CD13⁺, ARG⁺/CD14⁺, CD34⁺/Lin⁻/DR⁻/CD11b⁺/CD15⁺ and CD14⁺/HLA-DR⁻/low; CD11b⁺/CD14⁻/CD15⁺, Lin⁻ /DR⁻, Lin⁻/DR⁻/CD33⁺, CD34⁺/CD33⁺/CD13⁺, ARG⁺/CD14⁺, CD34⁺/Lin⁻/DR⁻ /CD11b⁺/CD15⁺ and Lin⁻/HLA-DR⁻/low/CD11b⁺/CD33⁺; CD11b⁺/CD14⁻/CD15⁺, Lin⁻/DR⁻ /CD33⁺, CD34⁺/CD33⁺/CD13⁺, ARG⁺/CD14⁺, CD34⁺/Lin⁻/DR⁻/CD11b⁺/CD15⁺, CD14⁺/HLA-DR⁻/low and Lin⁻/HLA-DR⁻/low/CD11b⁺/CD33⁺; CD11b⁺/CD14⁺/CD15⁺, Lin⁻/DR⁻, Lin⁻/DR⁻/CD33⁺, CD34⁺/CD33⁺/CD13⁺, ARG⁺/CD14⁺, CD34⁺/Lin⁻/DR⁻ /CD11b⁺/CD15⁺ and CD14⁺/HLA-DR⁻/low; CD11b⁺/CD14⁺/CD15⁺, Lin⁻/DR⁻, Lin⁻/DR⁻ /CD33⁺, CD34⁺/CD33⁺/CD13⁺, ARG⁺/CD14⁺, CD34⁺/Lin⁻/DR⁻/CD11b⁺/CD15⁺ and Lin⁻ /HLA-DR⁻/low/CD11b⁺/CD33⁺; CD11b⁺/CD14⁺/CD15⁺, Lin⁻/DR⁻/CD33⁺, CD34⁺/CD33⁺/CD13⁺, ARG⁺/CD14⁺, CD34⁺/Lin⁻/DR⁻/CD11b⁺/CD15⁺, CD14⁺/HLA-DR⁻ /low and Lin⁻/HLA-DR⁻/low/CD11b⁺/CD33⁺; or Lin⁻/DR⁻, Lin⁻/DR⁻/CD33⁺, CD34⁺/CD33⁺/CD13⁺, ARG⁺/CD14⁺, CD34⁺/Lin⁻/DR⁻/CD11b⁺/CD15⁺, CD14⁺/HLA-DR⁻ /low and Lin⁻/HLA-DR⁻/low/CD11b⁺/CD33⁺.

In certain embodiments, the cells whose levels are measured in step (i) of the method of the invention are γδ T-cells and any eight cell types of the MDSCs listed, i.e., CD11b⁺/CD14⁻, CD11b⁺/CD14⁻/CD15⁺, CD11b⁺/CD14⁺/CD15⁺, Lin⁻/DR⁻, Lin⁻/DR⁻ /CD33⁺, CD34⁺/CD33⁺/CD13⁺, ARG⁺/CD14⁺ and CD34⁺/Lin⁻/DR⁻/CD11b⁺/CD15⁺; CD11b⁺/CD14⁻, CD11b⁺/CD14⁻/CD15⁺, CD11b⁺/CD14⁺/CD15⁺, Lin⁻/DR⁻, Lin⁻/DR⁻ /CD33⁺, CD34⁺/CD33⁺/CD13⁺, ARG⁺/CD14⁺ and CD14⁺/HLA-DR⁻/low; CD11b⁺/CD14⁻, CD11b⁺/CD14⁻/CD15⁺, CD11b⁺/CD14⁺/CD15⁺, Lin⁻/DR⁻, Lin⁻/DR⁻/CD33⁺, CD34⁺/CD33⁺/CD13⁺, ARG⁺/CD14⁺ and Lin⁻/HLA-DR⁻/low/CD11b⁺/CD33⁺; CD11b⁺/CD14⁻, CD11b⁺/CD14⁺/CD15⁺, Lin⁻/DR⁻, Lin⁻/DR⁻/CD33⁺, CD34⁺/CD33⁺/CD13⁺, ARG⁺/CD14⁺, CD34⁺/Lin⁻/DR⁻/CD11b⁺/CD15⁺ and CD14⁺/HLA-DR⁻/low; CD11b⁺/CD14⁻, CD11b⁺/CD14⁺/CD15⁺, Lin⁻/DR⁻, Lin⁻/DR⁻/CD33⁺, CD34⁺/CD33⁺/CD13⁺, ARG⁺/CD14⁺, CD34⁺/Lin⁻/DR⁻/CD11b⁺/CD15⁺ and Lin⁻/HLA-DR⁻ /low/CD11b⁺/CD33⁺; or CD11b⁺/CD14⁻, Lin⁻/DR⁻, Lin⁻/DR⁻/CD33⁺, CD34⁺/CD33⁺/CD13⁺, ARG⁺/CD14⁺, CD34⁺/Lin⁻/DR⁻/CD11b⁺/CD15⁺, CD14⁺/HLA-DR⁻ /low and Lin⁻/HLA-DR⁻/low/CD11b⁺/CD33⁺.

In certain embodiments, the cells whose levels are measured in step (i) of the method of the invention are γδ T-cells and any nine cell types of the MDSCs listed, i.e., CD11b⁺/CD14⁻, CD11b⁺/CD14⁻/CD15⁺, CD11b⁺/CD14⁺/CD15⁺, Lin⁻/DR⁻, Lin⁻/DR⁻ /CD33⁺, CD34⁺/CD33⁺/CD13⁺, ARG⁺/CD14⁺, CD34⁺/Lin⁻/DR⁻/CD11b⁺/CD15⁺ and CD14⁺/HLA-DR⁻/low; CD11b⁺/CD14⁻, CD11b⁺/CD14⁻/CD15⁺, CD11b⁺/CD14⁺/CD15⁺, Lin⁻/DR⁻, Lin⁻/DR⁻/CD33⁺, CD34⁺/CD33⁺/CD13⁺, ARG⁺/CD14⁺, CD34⁺/Lin⁻/DR⁻ /CD11b⁺/CD15⁺ and Lin⁻/HLA-DK/low/CD11b⁺/CD33⁺; or CD11b⁺/CD14⁻, CD11b⁺/CD14⁺/CD15⁺, Lin⁻/DR⁻, Lin⁻/DR⁻/CD33⁺, CD34⁺/CD33⁺/CD13⁺, ARG⁺/CD14⁺, CD34⁺/Lin⁻/DR⁻/CD11b⁺/CD15⁺, CD14⁺/HLA-DR⁻/low and Lin⁻/HLA-DR⁻ /low/CD11b⁺/CD33⁺.

In certain embodiments, the cells whose levels are measured in step (i) of the method of the invention are γδ T-cells and all the ten cell types of the MDSCs listed, i.e., CD11b⁺/CD14⁻, CD11b⁺/CD14⁻/CD15⁺, CD11b⁺/CD14⁺/CD15⁺, Lin⁻/DR⁻, Lin⁻/DR⁻ /CD33⁺, CD34⁺/CD33⁺/CD13⁺, ARG⁺/CD14⁺, CD34⁺/Lin⁻/DR⁻/CD11b⁺/CD15⁺, CD14⁺/HLA-DR⁻/low and Lin⁻/HLA-DR⁻/low/CD11b⁺/CD33⁺.

In a particular embodiment, the cells whose levels are measured in step (i) of the method of the present invention are γδ T-cells and CD11b⁺/CD14⁻/CD15⁺.

In certain embodiments, the present invention relates to a method for diagnosing the likelihood of AD in a tested individual as defined above, when further comprising measuring in step (i) the level of at least one cell type of pro-inflammatory monocytes in said blood sample; and comparing in step (ii) the level of said at least one cell type of pro-inflammatory monocytes with a reference level representing a range level of said at least one cell type of pro-inflammatory monocytes in blood samples of age-matched controls, wherein an increase in the level of γδ T-cells; no change in the level of each one of said at least one cell type of MDSCs; and an increase in the level of at least one of said at least one cell type of pro-inflammatory monocytes indicate that said individual has a higher likelihood of having AD than said age-matched controls.

The term "pro-inflammatory monocytes", as used herein, refers to a non-classical type of monocytes characterized by low-level expression of CD14 and additional co-expression of the CD16 receptor (CD14⁺/CD16⁺ monocytes), which develop from the CD14⁺⁺ monocytes.

In particular such embodiments, the cells whose levels are measured in step (i) of the method of the invention are thus γδ T-cells; at least one cell type of the MDSCs listed above; and CD14⁺/CD16⁺ cells. More particular such embodiments are those wherein a sole cell type of MDSCs is measured, or those wherein any combination of two, three, four, five, six, or more cell types of MDCSs as defined above are measured.

In one particular embodiment exemplified herein, the cells whose levels are measured in step (i) of the method of the present invention are γδ T-cells, CD11b⁺/CD14⁻ /CD15⁺ cells and CD14⁺/CD16⁺ cells, wherein an increase in the level of γδ T-cells; no change in the level of CD11b⁺/CD14⁻/CD15⁺ cells; and an increase in the level of CD14⁺/CD16⁺ cells indicate that said individual has a higher likelihood of having AD than said age-matched controls.

In a particular such aspect, the present invention thus relates to a method for diagnosing the likelihood of AD in a tested individual, said method comprising:
(i) measuring the levels of γδ T-cells, CD11b⁺/CD14⁻/CD15⁺ cells and CD14⁺/CD16⁺ cells in a peripheral blood sample obtained from said individual; and
(ii) comparing the levels measured in (i) with reference levels representing range levels of γδ T-cells, CD11b⁺/CD14⁻/CD15⁺ cells and CD14⁺/CD16⁺ cells, respectively, in blood samples of age-matched controls, thus obtaining a profile expressing the levels measured in (i) relative to said reference levels, respectively,
wherein an increase in the level of γδ T-cells; no change in the level of CD11b⁺/CD14⁻/CD15⁺ cells; and an increase in the level of CD14⁺/CD16⁺ cells indicate that said individual has a higher likelihood of having AD than said age-matched controls.

In certain embodiments, the present invention relates to a method as defined above, wherein the cell types whose levels are measured in step (i) are γδ T-cells, CD11b⁺/CD14⁻/CD15⁺ cells and CD14⁺/CD16⁺ cells, and the profile obtained in step (ii), expressing the level measured in step (i) for each one of the cell types and indicating a higher likelihood of AD for the tested individual, includes increase of at least 50%, at least 60%, at least 70%, at least 80%, at least 90%, about 100%, or more, preferably about 100%, in the level of γδ T-cells in the blood sample analyzed, i.e., the blood sample obtained from the tested individual, compared with a reference level representing a range level of γδ T-cells in blood samples of age-matched controls; and increase of at least 30%, at least 35%, at least 40%, at least 45%, about 50%, or more, preferably about 50%, in the level of CD14⁺/CD16⁺ cells in the blood sample analyzed compared with a reference level representing a range level of CD14⁺/CD16⁺ cells in blood samples of age-matched controls.

The peripheral blood sample analyzed in step (i) of the method of the present invention is obtained by taking blood sample from the individual being diagnosed for the likelihood of AD; and contacting said blood sample with various types of antibodies each directed to one of the cell types or subsets whose levels are measured, i.e., γδ T-cells, at least one cell type of MDSCs as defined above, and optionally at least one cell type of pro-inflammatory monocytes as defined above, wherein each type of the antibodies used is either directly or indirectly labeled with, e.g., a fluorescent marker. The level of each one of the cell types or subsets is then measured in said blood sample utilizing any suitable technique known in the art, preferably by FACS as described in the Examples section hereinafter.

The level measured for each one of the cell types or subsets tested, according to step (i) of the diagnosing method of the invention, is compared with a reference level representing a range level of said cell type or subset in blood samples of age-matched controls, i.e., a group of healthy individuals in the same age-group as the tested individual. This range level, also termed herein "the normal range level", is derived from the available medical knowledge and represents the normal range level for the specific cell type or subset tested in blood samples of age-matched controls.

According to step (ii) of this method, after comparing the level measured for each one of the cell types or subsets tested with the reference level, i.e., the normal range level, thereof, a profile is obtained, expressing the level of each one of the cell types of subsets tested in the blood sample obtained from the tested individual relative to the level of each one of these cell types or subsets, respectively, in blood samples of age-matched controls.

The profile obtained in step (ii) of the diagnosing method of the invention is a relative profile, showing the level of each one of the cell types or subsets measured according to this method in the blood sample obtained from the tested individual relative to the reference level of said cell type or subset in blood samples of age-matched controls. Since the reference level to which the measured level is compared represents, in fact, a range level of said cell type or subset in blood samples of healthy individuals in the same age-group as the tested individual, each one of the levels measured in the blood sample tested can be compared with either the median value or the upper level value, but preferably with the upper level value, of the normal reference.

According to step (i) of this method as defined above, the level of γδ T-cells, at least one cell type of MDSCs, and optionally at least one cell type of a pro-inflammatory monocytes, are measured, and therefore, the profile obtained in step (ii) expresses the level of at least two, i.e., two, three, four, five, six, seven, eight, or more, but preferably three or more cell types or subsets, as defined above.

The relative level of each one of the cell types or subsets measured is represented in the profile by "increase", indicating that the level of said cell type or subset in the blood sample tested is increased compared with the upper limit of the normal range level thereof, i.e., the range level of said cell type or subset in blood samples of age-matched controls, by at least about 30%, 35%, 40%, 45%, 50%, 55%, 60%, 65%, 70%, 75%, 80%, 85%, 90%, 95%, 100%, or more; "decrease", indicating that the level of said cell type or subset in the blood sample tested is decreased compared with the lower limit of the normal range level thereof by at least about 10%, 15%, 20%, 25%, 30%, 35%, 40%, 45%, 50%, or more; or "no change", indicating that the level of said cell type or subset in the blood sample tested is neither increased nor decreased as defined above, i.e., within or close to the normal range level thereof.

AD develops for an unknown and variable amount of time before becoming fully apparent, and it can progress undiagnosed for years. Moreover, early symptoms of AD are often mistakenly thought to be "age-related" concerns, or manifestations of stress. AD is usually diagnosed clinically from the patient history, collateral history from relatives, and clinical observations, based on the presence of characteristic neurological and neuropsychological features and the absence of alterbative conditions. The method discussed above is aimed at diagnosing, more specifically early diagnosing, the likelihood of AD in a tested individual, wherein the individuals subjected to this method are those exhibiting certain signs that might be associated with AD, particularly difficulty in remembering recent events (rather than older memories of the person's life, also called "episodic memory", facts learned, i.e., semantic memory, and implicit memory, i.e., the memory of the body on how to do things, which are affected to a lesser degree), which is the most common symptom in early stages of the disease.

Individuals diagnosed according to the method of the present invention as having a higher likelihood of AD, can be directed to subsequent confirmatory diagnosis steps, or may start receiving a therapeutic treatment aimed at treating the cognitive manifestations of AD, e.g., an acetylcholineesterase inhibitor such as tacrine, rivastigmine, galantamine and donepezil, or an N-methyl d-aspartate (NMDA) receptor antagonist such as memantine, or psychosocial intervention. When AD is suspected, the diagnosis is usually confirmed with tests that evaluate behaviour and thinking abilities, often followed by a brain scan, also called neuroimaging, if available. Advanced medical imaging with computed tomography (CT) or magnetic resonance imaging (MRI), and with single photon emission computed tomography (SPECT) or positron emission tomography (PET) can be used to exclude other cerebral pathology or subtypes of dementia. The decision whether subsequent confirmatory diagnosis steps are required, or a treatment can be provided, will be determined as deemed appropriate by the practitioner.

The phrase "a range level", as used herein with respect to a particular cell type or subset in blood samples of age-matched controls, refers to the normal range level for a specific cell type or subset in blood samples of age-matched controls, as defined above.

As described above, in contrast to certain neurodegenerative diseases such as ALS, no alteration was observed in the level of the MDSCs CD11b⁺/CD14⁻/CD15⁺ in peripheral blood samples of Alzheimer's patients compared with the normal range level of these cells. Therefore, the level of these monocytes can be used, in combinations with the level of other cell types or subsets as defined above, for diagnosing the likelihood of AD in a tested individual.

In a further aspect, the present invention provides a kit for use in diagnosing the likelihood of AD in a tested individual, said kit comprising:
(i) antibodies against each one of cell types selected from gamma-delta (γδ) T-cells and at least one cell type of myeloid derived suppressor cells (MDSCs); and
(ii) reagents for detecting said antibodies.

The kit of the present invention can be used for carrying out the non-therapeutic method described above, i.e., the method in which the likelihood of AD in a tested individual is diagnosed.

In certain embodiments, the kit of the present invention is used for diagnosing the likelihood of AD in a tested individual, as defined above, wherein said cell types further include at least one cell type of pro-inflammatory monocytes as defined above. In particular such embodiments, said pro-inflammatory monocytes are CD14⁺/CD16⁺ cells.

The kit of the invention further comprises antibodies against each one of said cell types, as well as reagents required for the detection of those antibodies. The antibodies may be either monoclonal or polyclonal, but they are preferably monoclonal antibodies. Both the antibodies and the reagents provided are used for measuring the levels of the cell types listed, in said blood sample.

As defined by the non-therapeutic methods of the invention, the level measured for each one of the cell types is compared with a range level of said cell type in blood samples of age-matched controls so as to evaluate whether the level measured is higher than, or within, the normal range level of said cell type, i.e., the range level of said cell type in blood samples of age-matched controls. These data are compared with reference levels, further included in the kit, expressing range levels of said cell types in blood samples of age-matched controls, so as to determine whether said individual has a higher likelihood of having AD than said age-matched controls.

The invention will now be illustrated by the following non-limiting Examples.

### EXAMPLES

### Materials and Methods

***Patients***: The patient's group included individuals, both males and females, which have been clinically diagnosed as suffering from AD and agreed to sign on the informed consent. The control group included male and female volunteers without clinical symptoms of AD, who agreed to sign on the informed consent. Alzheimer's patients and controls that were included into the study have been examined for their cognitive skills using the mini-mental test. A blood sample of up to 20 ml was taken and delivered to the lab to be analyzed for the different cellular components after excluding the presence of the following viruses: HCV, HBSAG, HIV, HTLV and TPHA. Blood analysis was performed between 18-24 hours from the time it was taken.

***Whole blood FACS staining*:** 50 µl of whole blood samples were incubated with 5 µl of each of the designated mAb for 45 minutes at 4°C. Two ml of FACSlyse (Becton Dickinson, San Jose, CA) was added to each tube, and the tubes were then incubated at room temperature for 12 minutes, followed by wash with 2 ml PBS. From each sample, 10⁵ events were acquired by FACSCalibur (Becton Dickinson, San Jose, CA) and analyzed by the FCS Express V3 software.

***The designated mAb's*:** CD3, CD4, CD8, CD14, CD15, CD11b, CD16, Lin, HLA-DR, CD33, TCRgd - Becton Dickinson, San Jose, CA. TLR4 eBioscience San Diego, CA.

### Example 1. Accuracy and robustness of results in healthy volunteers

In this study, the distribution of single markers on total live peripheral blood mononuclear cells (PBMC) was tested, first using blood from young, healthy volunteers, so as to examine the accuracy and robustness of our measurements, and then in a controlled study comparing Alzheimer's patients and matched age controls.

Freshly isolated PBMC of healthy volunteers were stained with fluorescein isothiocyanate (FITC), phycoerythrin (PE) or allophycocyanin (APC)-labeled mononuclear antibodies against CD3, CD14, CD19, CD11c, CD34 and CD15, and the proportion and level of expression of each one of these markers were analyzed by fluorescence-activated cell sorting (FACS) (**Figs. 1A-1B**).

Freshly isolated PBMC of healthy volunteers were double stained with APC-labeled mononuclear antibodies against CD3 and either FITC- or PE-labeled mononuclear antibodies against CD4, CD8, CTLA4 or TCRgd, and the percentage of cells expressing each one of these markers out of the CD3 positive cell population, as well as the intensity of expression, were measured (**Table 1;** **Figs. 2A-2B**).

The results shown in **Figs. 1-2** indicate that the markers with relatively small deviations between the different blood samples can be monitored. The results presented in Table 1 show that the distribution of CD14 and CD16 on peripheral blood monocytes can be separated into three distinct subpopulations: high, dim and negative expression of CD14, each represent a different cellular phenotype.

**Table 1: Monocyte's sub-population**

| | **CD14⁺ CD40⁺** | **CD 14high CD16⁻** | **CD14high CD16⁺** | **CD14dim CD16⁺** | **CD14⁻CD15⁺ CD11b⁺** |
|---|---|---|---|---|---|
| **% of positive cells** | 40.27 | 75.23 | 10.38 | 4.44 | 15.82 |
| | (4.72) | (4.16) | (3.71) | (0.62) | (2.81) |
| | n=4 | n=4 | n=4 | n=4 | n=4 |
| **Mean fluorescence intensity** | 213.38 | 247.29 | 167.42 | 32.64 | 78.05 |
| | (17.45) | (44.64) | (27.99) | (1.78) | (20.97) |

### Example 2. Alzheimer's patients show elevated level of both γδ-T cells and CD14⁺/CD16⁺ cells in PBMC compared with healthy controls

The studies described herein were conducted using about 32 blood samples, about half of them obtained from Alzheimer's patients and half of them obtained from age-matched healthy volunteers. In addition, 7 blood samples of amyotrophic lateral sclerosis (ALS) patients, another neurodegenerative disease, were analyzed. All blood samples were encoded, and analysis of the results was done blindly.

**Table 2: Differential count of peripheral mononuclear cells (% of total PBMC)**

| | | Average | SD | Min | Median | Max | n |
|---|---|---|---|---|---|---|---|
| **Monocytes CD14** | **Healthy** | 16.6 | 6.28 | 9.1 | 16.2 | 29.8 | 14 |
| | **AD** | 19.1 | 4.95 | 11.6 | 18.5 | 29.9 | 15 |
| | **ALS** | 18.9 | 4.3 | 13.4 | 17.3 | 25.7 | 7 |
| **T-cells CD3** | **Healthy** | 53.9 | 11.95 | 27.7 | 57.6 | 69.7 | 14 |
| | **AD** | 57.5 | 9.39 | 40.4 | 56.5 | 73.2 | 16 |
| | **ALS** | 49.4 | 8.4 | 38.3 | 48 | 63.1 | 7 |
| **B-cells CD19** | **Healthy** | 8.2 | 3.42 | 2.8 | 8.0 | 17.0 | 14 |
| | **AD** | 7.2 | 3.44 | 2.9 | 5.7 | 13.0 | 16 |
| | **ALS** | ND | ND | ND | ND | ND | ND |

The proportion of PBMC population as measured by flow cytometry (monocytes-CD14, T-cells-CD3 and B-cells-CD19) are presented in **Table 2,** indicating no significant difference between the patients and the healthy controls.

The percentage of T-helper (CD4 positive cells) and cytotoxic-T cells (CD8 positive cells) out of total T-cells (CD3 positive cells), as well as the ratio between these two cell-populations, were measured using flow cytometey method in the blood of the patients and healthy controls. No difference was found between the two groups (**Table 3**).

**Table 3: Leukocyte sub-populations**

| | | Average | SD | Min | Median | Max | n |
|---|---|---|---|---|---|---|---|
| **Monocytes CD14** | **Healthy** | 64.3 | 17.13 | 20.9 | 68.4 | 86.9 | 14 |
| | **AD** | 63.5 | 16.37 | 33.6 | 66.1 | 87.1 | 16 |
| | **ALS** | 65.5 | 5.2 | 59.3 | 65.2 | 75.4 | 7 |
| **T-cells CD3** | **Healthy** | 31.7 | 16.47 | 11.7 | 29.3 | 78.2 | 14 |
| | **AD** | 30.5 | 14.59 | 11.3 | 29.7 | 56.6 | 16 |
| | **ALS** | 27.9 | 7.8 | 16.1 | 31.5 | 34.9 | 7 |
| **B-cells CD19** | **Healthy** | 2.8 | 1.96 | 0.3 | 2.3 | 8.1 | 14 |
| | **AD** | 3.0 | 2.15 | 0.6 | 2.2 | 7.7 | 16 |
| | **ALS** | 2.6 | 1.1 | 1.7 | 2.0 | 4.3 | 7 |

While no differences were found in the amount of lymphocytes and monocytes in the blood of AD patients, ALS and healthy volunteers, as shown above, significant differences in sub-population of lymphocytes and monocytes were found, as shown in **Table 4.** These cell types are typically involved with regulation of the adaptive immune response as described below.

**Table 4: Percentage of sub-populations out of total lymphocytes and monocytes respectively**

| | | Average | SD | Min | Median | Max | n |
|---|---|---|---|---|---|---|---|
| **γδ-T-cells** | **Healthy** | 2.6 | 1.96 | 0.7 | 1.9 | 7.3 | 14 |
| | **AD** | 6.0 | 2.87 | 2.3 | 5.0 | 12.8 | 16 |
| | **ALS** | 12.9 | 8.1 | 1.8 | 11.8 | 26.8 | 7 |
| **CD14⁺/CD16⁺** | **Healthy** | 10.5 | 5.78 | 2.4 | 9.7 | 20.4 | 14 |
| | **AD** | 16.3 | 8.70 | 3.2 | 17.4 | 34.9 | 16 |
| | **ALS** | 7.8 | 3.9 | 2.5 | 8.1 | 14.7 | 7 |
| **MDSC** | **Healthy** | 1.9 | 2.0 | 0.04 | 1.05 | 6.94 | 14 |
| | **AD** | 1.7 | 1.8 | 0.2 | 0.9 | 5.8 | 16 |
| | **ALS** | 11.0 | 10.5 | 1.4 | 9.2 | 32.6 | 7 |

Gamma-delta (γδ)-T cells were found to be significantly elevated in the AD patients in comparison to the healthy controls, but less than in the ALS patients. This group of cells has a complex behavior; they were shown to act as "first line of defense", "regulatory cells", and as "bridge between innate and adaptive responses". Their exact role in the pathological cascade of AD should be further investigated. Yet, the preliminary results suggest that they may be used for AD diagnosis with relatively high accuracy.

Elevated levels of the pro-inflammatory sub-set of monocytes (CD14⁺/CD16⁺) were found in the AD patients but not in the ALS patients. More particularly, while in the healthy donors these cells accounted for about 10% of all monocytes, in the AD patients they accounted for about 16% of all monocytes. The CD14⁺/CD16⁺ cells have been shown to efficiently produce the pro-inflammatory cytokine TNFα, while they produce no or little of the anti-inflammatory cytokine IL-10 (Belge *et al*., 2002). This may dictate the phenotype of the adaptive immune response towards a Th1 type of response instead of the beneficial Th2 response. It is thus important to examine the correlation between the level of the cells and the severity of the disease.

Within the monocyte population, while a dramatic elevation was found in the percentage of cells with the markers CD14⁻/CD11b⁺/CD15⁺, a phenotype associated with myeloid-derived suppressor cells (MDSCs), in the blood of patients with ALS, no difference in the percentage of these cells was found between the AD patients and the healthy controls. These cells constitute a population of immature myeloid cells with potent immunosuppressive functions.

The significant differences described above in white blood cells profile of AD patients in comparison to age-matched controls and to ALS patients can be used for accurate diagnosis of AD.

An analysis was performed so as to evaluate the potential of the findings described herein to be used as biomarkers for accurate diagnosis of AD. The analysis included the evaluation of the above-described independent immune system antigens, coupled with a sophisticated analytical algorithm for data processing in an effort to clearly define the molecular relationship of these antigens and the test's performance in regard with AD diagnosis. At this stage, and to evaluate the potential of the individual markers, per each of the above described markers, we determined meaningful results based on accuracy levels. In particular, we examined the maximal sum of sensitivity and specificity and area under the receiver operator characteristic curve (AUC of ROC). AUC is an overall measure of the accuracy of a test. As a general rule, with exceptions, AUC should be at about 0.8 or higher before a marker is considered feasible.

The ROC curve for γδ-T-cells is shown in **Fig. 3**. This marker appears to be highly sensitive and accurate for AD diagnosis, as shown in **Table 5**. It may be expected that the combination of this marker with the additional markers, will contribute to the sensitivity, selectivity and specificity of this test. Furthermore, it is suggested that the level of these cells may also correlate with disease severity.

**Table 5: The sensitivity and specificity of the biomarkers found for diagnosis of AD**

| | **Sensitivity** | **Specificity** | **AUC** | ***P*** |
|---|---|---|---|---|
| **γδ-T-cells** | 87% | 85% | 0.87 | <0.001 |
| **CD14⁺/CD16⁺** | 60% | 85% | 0.70 | 0.064 |

### REFERENCES

Avidan H., Kipnis J., Butovsky O., Caspi R.R., Schwartz M., Vaccination with autoantigen protects against aggregated beta-amyloid and glutamate toxicity by controlling microglia: effect of CD4+CD25+ T cells. Eur JImmunol., 2004, 34(12), 3434-3445
Aw D., Silva A.B., Palmer D.B., Immunosenescence: emerging challenges for an ageing population. Immunology, 2007, 120(4), 435-446
Beers D.R., Henkel J.S., Zhao W., Wang J., Appel S.H., CD4+ T cells support glial neuroprotection, slow disease progression, and modify glial morphology in an animal model of inherited ALS, Proc Natl Acad Sci USA, 2008, 105(40), 15558-15563
Belge K.U., Dayyani F., Horelt A., Siedlar M., Frankenberger M., Frankenberger B., Espevik T., Ziegler Heitbrock L., The proinflammatory CD14+CD16+DR++ monocytes are a major source of TNF. J Immunol., 2002, 168(7), 3536-3542
Brandes M., Willimann K., Bioley G., Lévy N., Eberl M., Luo M., Tampé R., Lévy F., Romero P., Moser B., Cross-presenting human gammadelta T cells induce robust CD8+ alphabeta T cell responses, Proc Natl Acad Sci USA, 2009, 106(7), 2307-2312
Butovsky O., Ziv Y., Schwartz A., Landa G., Talpalar A.E., Pluchino S., Martino G., Schwartz M., Mol Cell Neurosci., 2006a, 31(1), 149-160 3
Butovsky O., Koronyo-Hamaoui M., Kunis G., Ophir E., Landa G., Cohen H., Schwartz M., Glatiramer acetate fights against Alzheimer's disease by inducing dendritic-like microglia expressing insulin-like growth factor 1. Proc Natl Acad Sci USA., 2006b, 103(31) 29
Butovsky O., Kunis G., Koronyo-Hamaoui M., Schwartz M., Selective ablation of bone marrow-derived dendritic cells increases amyloid plaques in a mouse Alzheimer's disease model. Eur J Neurosci., 2007, 26(2), 413-416
Chiu I.M., Chen A., Zheng Y., Kosaras B., Tsiftsoglou S.A., Vartanian T.K., Brown R.H. Jr, Carroll M.C., T lymphocytes potentiate endogenous neuroprotective inflammation in a mouse model of ALS, Proc Natl Acad Sci USA, 2008, 105(46), 17913-17918
Ekdahl C.T., Kokaia Z., Lindvall O., Brain inflammation and adult neurogenesis: The dual role of microglia. Neuroscience, 2009, 158(3), 1021-1029. Epub **2008**
Girardi M., Immunosurveillance and immunoregulation by gammadelta T cells, J Invest Dermatol., 2006, 126(1), 25-31
Hauben E., Nevo U., Yoles E., Moalem G., Agranov E., Mor F., Akselrod S., Neeman M., Cohen I.R., Schwartz M., Lancet, 2000, 355(9200), 286-287
Hauben E., Gothilf A., Cohen A., Butovsky O., Nevo U., Smirnov I., Yoles E., Akselrod S., Schwartz M., J Neurosci., 2003, 23(25), 8808-8819
Kipnis J., Yoles E., Schori H., Hauben E., Shaked I., Schwartz M., J Neurosci., 2001, 21(13), 4564-4571
Kipnis J., Mizrahi T., Hauben E., Shaked I., Shevach E., Schwartz M., Proc Natl Acad Sci USA, 2002, 99(24), 15620-15625
Kipnis J., Cohen H., Cardon M., Ziv Y., Schwartz M., Proc Natl Acad Sci USA, 2004, 101(21), 8180-8185
Kipnis J., Schwartz M., Controlled autoimmunity in CNS maintenance and repair: naturally occurring CD4+CD25+ regulatory T-Cells at the crossroads of health and disease. Neuromolecular Med., 2005, 7(3), 197-206
Mizrahi T., Hauben E., Schwartz M., The tissue-specific self-pathogen is the protective self-antigen: the case of uveitis. J Immunol., 2002, 169(10), 5971-5977
Moalem G., Leibowitz-Amit R., Yoles E., Mor F., Cohen I.R., Schwartz M., Nat Med., 1999, 5(1), 49-55
Nevo U., Kipnis J., Golding I., Shaked I., Neumann A., Akselrod S., Schwartz M., Autoimmunity as a special case of immunity: removing threats from within. Trends Mol Med., 2003, 9(3), 88-93
Nevo U., Golding I., Neumann A.U., Schwartz M., Akselrod S., Autoimmunity as an immune defense against degenerative processes: a primary mathematical model illustrating the bright side of autoimmunity. J Theor Biol., 2004, 227(4), 583-592
Nichol K.E, Poon W.W., Parachikova A.I., Cribbs D.H., Glabe C.G., Cotman C.W., Exercise alters the immune profile in Tg2576 Alzheimer mice toward a response coincident with improved cognitive performance and decreased amyloid. J Neuroinflammation, 2008, 5, 13
Ochoa A.C., Zea A.H., Hernandez C., Rodriguez P.C., Arginase, prostaglandins, and myeloid-derived suppressor cells in renal cell carcinoma, Clin Cancer Res., 2007, 13(2 Pt 2), 721s-726s
Rapalino O., Lazarov-Spiegler O., Agranov E., Velan G.J., Yoles E., Fraidakis M., Solomon A., Gepstein R., Katz A., Belkin M., Hadani M., Schwartz M., Nat Med., 1998, 4(7), 814-821
Roark C.L., Simonian P.L., Fontenot A.P., Born W.K., O'Brien R.L., Gammadelta T cells: an important source of IL-17, Curr Opin Immunol., 2008, 20(3), 353-357
Ron-Harel N., Schwartz M., Immune senescence and brain aging: can rejuvenation of immunity reverse memory loss? Trends Neurosci., 2009, 32(7), 367-375
Schori H., Yoles E., Schwartz M., T-cell-based immunity counteracts the potential toxicity of glutamate in the central nervous system. J Neuroimmunol., 2001, 119(2), 199-204
Schwartz M., Physiological approaches to neuroprotection. boosting of protective autoimmunity. Surv Ophthalmol., 2001, 45 Suppl 3, S256-60
Schwartz M., Hauben E., Differing views on spinal cord repair. Science, 2002, 296(5572), 1400
Schwartz M., Kipnis J., Autoimmunity on alert: naturally occurring regulatory CD4(+)CD25(+) T cells as part of the evolutionary compromise between a 'need' and a 'risk'. Trends Immunol., 2002, 23(11), 530-534
Schwartz M., Shaked I., Fisher J., Mizrahi T., Schori H., Trends Neurosci., 2003, 26(6), 297-302
Schwartz M., Kipnis J., Self and non-self discrimination is needed for the existence rather than deletion of autoimmunity: the role of regulatory T cells in protective autoimmunity. Cell Mol Life Sci., 2004, 61(18), 2285-2289
Schwartz M., Ziv Y., Immunity to self and self-maintenance: a unified theory of brain pathologies. Trends Immunol., 2008a, 29(5), 211-219
Schwartz M., Ziv Y., Immunity to self and self-maintenance: what can tumor immunology teach us about ALS and Alzheimer's disease? Trends Pharmacol Sci., 2008b, 29(6), 287-293
Shaked I., Porat Z., Gersner R., Kipnis J., Schwartz M., J Neuroimmunol., 2004, 146(1-2), 84-93
Shaked I., Tchoresh D., Gersner R., Meiri G., Mordechai S., Xiao X., Hart R.P., Schwartz M., JNeurochem., 2005, 92(5), 997-1009
Taams L.S., Akbar A.N., Curr Top Microbiol Immunol., 2005, 293, 115-131
Yoles E., Hauben E., Palgi O., Agranov E., Gothilf A., Cohen A., Kuchroo V., Cohen I.R., Weiner H., Schwartz M., Protective autoimmunity is a physiological response to CNS trauma. J Neurosci., 2001, 21(11), 3740-3748
Zea A.H., Rodriguez P.C., Atkins M.B., Hernandez C., Signoretti S., Zabaleta J., McDermott D., Quiceno D., Youmans A., O'Neill A., Mier J., Ochoa A.C., Arginase-producing myeloid suppressor cells in renal cell carcinoma patients: a mechanism of tumor evasion, Cancer Res., 2005, 65(8), 3044-3048
Ziv Y., Ron N., Butovsky O., Landa G., Sudai E., Greenberg N., Cohen H., Kipnis J., Schwartz M., Immune cells contribute to the maintenance of neurogenesis and spatial learning abilities in adulthood. Nat Neurosci., 2006, 9(2), 268-275
Ziv Y., Schwartz M., Immune-based regulation of adult neurogenesis: implications for learning and memory. Brain Behav Immun., 2008, 22(2), 167-176

## Claims

1. A method for diagnosing the likelihood of Alzheimer's disease (AD) in a tested individual, comprising:
(i) measuring the levels of gamma-delta (γδ) T-cells and at least one cell type of myeloid derived suppressor cells (MDSCs) in a peripheral blood sample obtained from said individual; and
(ii) comparing the levels measured in (i) with reference levels representing range levels of γδ T-cells and said at least one cell type of MDSCs, respectively, in blood samples of age-matched controls, thus obtaining a profile expressing the levels measured in (i) relative to said reference levels, respectively,
wherein an increase in the level of γδ T-cells; and no change in the level of each one of said at least one cell type of MDSCs indicate that said individual has a higher likelihood of having AD than said age-matched controls.

2. The method of claim 1, wherein said at least one cell type of MDSCs is CD11b⁺/CD14⁻, CD11b⁺/CD14⁻/CD15⁺, CD11b⁺/CD14⁺/CD15⁺, Lin⁻/DR⁻, Lin⁻/DR⁻ /CD33⁺, CD34⁺/CD33⁺/CD13⁺, ARG⁺/CD14⁺, CD34⁺/Lin⁻/DR⁻/CD11b⁺/CD15⁺, CD14⁺/HLA-DR⁻/low, or Lin⁻/HLA-DR⁻/low/CD11b⁺/CD33⁺ cells.

3. The method of claim 2, wherein said at least one cell type of MDSCs are CD11b⁺/CD14⁻/CD15⁺ cells.

4. The method of any one of claims 1 to 3, further comprising measuring in step (i) the level of at least one cell type of pro-inflammatory monocytes in said blood sample; and comparing in step (ii) the level of said at least one cell type of pro-inflammatory monocytes with a reference level representing a range level of said at least one cell type of pro-inflammatory monocytes in blood samples of age-matched controls,
wherein an increase in the level of γδ T-cells; no change in the level of each one of said at least one cell type of MDSCs; and an increase in the level of at least one of said at least one cell type of pro-inflammatory monocytes indicate that said individual has a higher likelihood of having AD than said age-matched controls.

5. The method of claim 4, wherein said at least one cell type of pro-inflammatory monocytes are CD14⁺/CD16⁺ cells.

6. The method of claim 4 or 5, wherein the levels of γδ T-cells, CD11b⁺/CD14⁻ /CD15⁺ cells and CD14⁺/CD16⁺ cells are measured in step (i).

7. The method of claim 6, wherein the level of γδ T-cells in the blood sample obtained from said individual is by at least 50%, 60%, 70%, 80%, 90%, or 100% higher than the level of γδ T-cells in blood samples of age-matched controls; and the level of CD14⁺/CD16⁺ cells in the blood sample obtained from said individual is by at least 30%, 35%, 40%, 45%, or 50% higher than the level of CD14⁺/CD16⁺ cells in blood samples of age-matched controls.

8. A kit for use in diagnosing the likelihood of Alzheimer's disease (AD) in a tested individual, said kit comprising:
(i) antibodies against each one of cell types selected from gamma-delta (γδ) T-cells and at least one cell type of myeloid derived suppressor cells (MDSCs); and
(ii) reagents for detecting said antibodies.

9. The kit for use of claim 8, wherein said cell types further include at least one cell type of pro-inflammatory monocytes.

10. The kit for use of claim 9, wherein said at least one cell type of pro-inflammatory monocytes are CD14⁺/CD16⁺ cells.

11. The kit for use of any one of claims 8-10, wherein said at least one cell type of MDSCs are CD11b⁺/CD14⁻/CD15⁺ cells.

## Patentansprüche

1. Verfahren zum Diagnostizieren der Wahrscheinlichkeit von Morbus Alzheimer (AD) bei einem getesteten Individuum, das umfasst:
(i) Messen der Spiegel von gamma-delta-T-Zellen (γδ-T-Zellen) und zumindest eines Zelltyps von myeloiden Suppressorzellen (MDSCs) in einer Probe peripheren Bluts des Individuums; und
(ii) Vergleichen der unter (i) gemessenen Spiegel mit Referenzspiegeln, die Bereichsspiegel von γδ-T-Zellen bzw. des zumindest einen Zelltyps von MDSCs darstellen, in Blutproben von altersabgeglichenen Kontrollen, wodurch ein Profil erhalten wird, das die unter (i) gemessenen Spiegel relativ zu den jeweiligen Referenzspiegeln ausdrückt,
wobei eine Erhöhung des Spiegels von γδ-T-Zellen; und keine Veränderung des Spiegels jedes des zumindest einen Zelltyps von MDSCs indizieren, dass bei dem Individuum eine höhere Wahrscheinlichkeit, dass es an AD leidet, als bei den altersabgeglichenen Kontrollen besteht.

2. Verfahren nach Anspruch 1, wobei der zumindest eine Zelltyp von MDSCs CD11b⁺/CD14⁻-, CD11b⁺/CD14⁻/CD15⁺-, CD11b⁺/CD14⁺/CD15⁺-, Lin⁻/DR⁻-, Lin⁻/DR⁻ /CD33⁺-, CD34⁺/CD33⁺/CD13⁺-, ARG⁺/CD14⁺-, CD34⁺/Lin⁻/DR⁻/CD11b⁺/CD15⁺-, CD14⁺/HLA-DR⁻/niedrig- oder Lin⁻/HLA-DR⁻/niedrig/CD11b⁺/CD33⁺-Zellen ist.

3. Verfahren nach Anspruch 2, wobei der zumindest eine Zelltyp von MDSCs CD11b⁺/CD14⁻/CD15⁺-Zellen ist.

4. Verfahren nach einem der Ansprüche 1 bis 3, das ferner in Schritt (i) das Messen des Spiegels zumindest eines Zelltyps von proinflammatorischen Monozyten in der Blutprobe; und in Schritt (ii) das Vergleichen des Spiegels des zumindest einen Zelltyps von proinflammatorischen Monozyten mit einem Referenzspiegel umfasst, der einen Bereichsspiegel des zumindest einen Zelltyps von proinflammatorischen Monozyten in Blutproben von altersabgeglichenen Kontrollen darstellt,
wobei eine Erhöhung des Spiegels von γδ-T-Zellen; keine Veränderung des Spiegels jedes des zumindest einen Zelltyps von MDSCs; und eine Erhöhung des Spiegels zumindest eines Zelltyps von proinflammatorischen Monozyten indizieren, dass bei dem Individuum eine höhere Wahrscheinlichkeit, dass es an AD leidet, als bei den altersabgeglichenen Kontrollen besteht.

5. Verfahren nach Anspruch 4, wobei der zumindest eine Zelltyp proinflammatorischer Monozyten CD14⁺/CD16⁺-Zellen ist.

6. Verfahren nach Anspruch 4 oder 5, wobei die Spiegel von γδ-T-Zellen, CD11b⁺/CD14⁻/CD15⁺-Zellen und CD14⁺/CD16⁺-Zellen in Schritt (i) gemessen werden.

7. Verfahren nach Anspruch 6, wobei der Spiegel von γδ-T-Zellen in der Blutprobe des Individuums um zumindest 50 %, 60 %, 70 %, 80 %, 90 % oder 100 % höher als der Spiegel von γδ-T-Zellen in Blutproben von altersabgeglichenen Kontrollen ist; und der Spiegel von CD14⁺/CD16⁺-Zellen in der Blutprobe des Individuums um zumindest 30 %, 35 %, 40 %, 45 % oder 50 % höher als der Spiegel von CD14⁺/CD16⁺-Zellen in Blutproben von altersabgeglichenen Kontrollen ist.

8. Verwendung eines Kits bei der Diagnose der Wahrscheinlichkeit von Morbus Alzheimer (AD) nach dem Verfahren von Anspruch 1 bei einem getesteten Individuum;
wobei der Kit umfasst:
(i) Antikörper gegen jeden von Zelltypen, die aus gamma-delta-T-Zellen (γδ-T-Zellen) und zumindest einem Zelltyp von myeloiden Suppressorzellen (MDSCs) ausgewählt sind; und
(ii) Reagenzien zum Nachweisen der Antikörper.

9. Verwendung nach Anspruch 8, wobei die Zelltypen ferner zumindest einen Zelltyp von proinflammatorischen Monozyten beinhalten.

10. Verwendung nach Anspruch 9, wobei der zumindest eine Zelltyp von proinflammatorischen Monozyten CD14⁺/CD16⁺-Zellen ist.

11. Verwendung nach einem der Ansprüche 8 bis 10, wobei der zumindest eine Zelltyp von MDSCs CD11b⁺/CD14⁻/CD15⁺-Zellen ist.

## Revendications

1. Procédé pour diagnostiquer la probabilité de maladie d'Alzheimer (MA) chez un individu testé, comprenant :
(i) la mesure des taux de cellules T gamma-delta (γδ) et d'au moins un type cellulaire de cellules myéloïdes suppressives (MDSC) dans un échantillon de sang périphérique obtenu chez ledit individu ; et
(ii) la comparaison des taux mesurés en (i) avec des taux de référence représentant des taux dans la plage des cellules T γδ et dudit au moins un type cellulaire de MDSC, respectivement, dans des échantillons de sang de contrôles de même âge, en obtenant ainsi un profil exprimant les taux mesurés en (i) par rapport auxdits taux de référence, respectivement,
dans lequel une augmentation du taux de cellules T γδ ; et aucune modification du taux de chacun dudit au moins un type cellulaire de MDSC indique que ledit individu présente une plus grande probabilité de présenter la MA que lesdits contrôles de même âge.

2. Procédé selon la revendication 1, dans lequel ledit au moins un type cellulaire de MDSC est des cellules CD11b⁺/CD14⁻, CD11b⁺/CD14⁻/CD15⁺, CD11b⁺/CD14⁺/CD15⁺, Lin⁻ /DR⁻, Lin⁻/DR⁻/CD33⁺, CD34⁺/CD33⁺/CD13⁺, ARG⁺/CD14^{+,} CD34⁺/Lin⁻/DR⁻/CD11b⁺/CD15⁺, CD14⁺/HLA-DR⁻/low, ou Lin⁻/HLA-DR⁻/low/CD11b⁺/CD33⁺.

3. Procédé selon la revendication 2, dans lequel ledit au moins un type cellulaire de MDSC est des cellules CD11b⁺/CD14⁻/CD15⁺.

4. Procédé selon l'une quelconque des revendications 1 à 3, comprenant en outre le mesure, à l'étape (i), du taux d'au moins un type cellulaire de monocytes pro-inflammatoires dans ledit échantillon de sang ; et la comparaison, à l'étape (ii), du taux dudit au moins un type cellulaire de monocytes pro-inflammatoires avec un taux de référence représentant un taux dans la plage dudit au moins un type cellulaire de monocytes pro-inflammatoires dans des échantillons de sang de contrôles de même âge,
dans lequel une augmentation du taux de cellules T γδ ; aucune modification du taux de chacun dudit au moins un type cellulaire de MDSC ; et une augmentation du taux d'au moins un dudit au moins un type cellulaire de monocytes pro-inflammatoires indique que ledit individu présente une plus grande probabilité de présenter la MA que lesdits contrôles de même âge.

5. Procédé selon la revendication 4, dans lequel ledit au moins un type cellulaire de monocytes pro-inflammatoires est des cellules CD14⁺/CD16⁺.

6. Procédé selon la revendication 4 ou 5, dans lequel les taux des cellules T γδ, des cellules CD11b⁺/CD14⁻/CD15⁺ et des cellules CD14⁺/CD16⁺ sont mesurés à l'étape (i).

7. Procédé selon la revendication 6, dans lequel le taux de cellules T γδ dans l'échantillon de sang obtenu chez ledit individu est au moins 50 %, 60 %, 70 %, 80 %, 90 % ou 100 % plus élevé que le taux de cellules T γδ dans des échantillons de sang de contrôles de même âge ; et le taux de cellules CD14⁺/CD16⁺ dans l'échantillon de sang obtenu chez ledit individu est au moins 30 %, 35 %, 40 %, 45 %, ou 50 % plus élevé que le taux de cellules CD14⁺/CD16⁺ dans des échantillons de sang de contrôles de même âge.

8. Utilisation d'un kit pour diagnostiquer la probabilité de maladie d'Alzheimer (MA) selon le procédé de la revendication 1 chez un individu testé, ledit kit comprenant :
(i) des anticorps dirigés contre chacun des types cellulaires sélectionnés parmi des cellules T gamma-delta (γδ) et au moins un type cellulaire de cellules myéloïdes suppressives (MDSC) ; et
(ii) des réactifs pour détecter lesdits anticorps.

9. Utilisation selon la revendication 8, dans laquelle lesdits types cellulaires comprennent en outre au moins un type cellulaire de monocytes pro-inflammatoires.

10. Utilisation selon la revendication 9, dans laquelle ledit au moins un type cellulaire de monocytes pro-inflammatoires est des cellules CD14⁺/CD16⁺.

11. Utilisation selon l'une quelconque des revendications 8-10, dans laquelle ledit au moins un type cellulaire de MDSC est des cellules CD11b⁺/CD14⁻/CD15⁺.
